(19) Europäisches Patentamt
European Patent Office
Office européen des brevets

(11) EP 4 306 132 A1

(12) **EUROPEAN PATENT APPLICATION**
published in accordance with Art. 153(4) EPC

(43) Date of publication:
**17.01.2024 Bulletin 2024/03**

(21) Application number: **22767451.2**

(22) Date of filing: **07.03.2022**

(51) International Patent Classification (IPC):
***A61K 48/00** (2006.01)* ***A61K 9/127** (2006.01)*
***A61K 39/00** (2006.01)* ***A61K 8/14** (2006.01)*
***A61K 8/60** (2006.01)* ***A61Q 19/00** (2006.01)*

(52) Cooperative Patent Classification (CPC):
**A61K 8/14; A61K 8/60; A61K 9/127; A61K 39/00; A61K 48/00; A61P 29/00; A61P 31/00; A61P 35/00; A61P 37/00; A61Q 19/00;** Y02A 50/30

(86) International application number:
**PCT/KR2022/003220**

(87) International publication number:
**WO 2022/191555 (15.09.2022 Gazette 2022/37)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **08.03.2021 KR 20210029929**

(71) Applicant: **Eyegene Inc.**
**Seoul 07528 (KR)**

(72) Inventors:
- **CHO, Yang Je**
  **Seoul 04423 (KR)**
- **KIM, Seok Hyun**
  **Goyang-si Gyeonggi-do 10416 (KR)**
- **KIM, Kwangsung**
  **Hanam-si Gyeonggi-do 12998 (KR)**
- **PARK, Shin Ae**
  **Ganghwa-gun Incheon 23048 (KR)**

(74) Representative: **J A Kemp LLP**
**80 Turnmill Street**
**London EC1M 5QU (GB)**

Remarks:
The complete document including Reference Table(s) and the Sequence Listing(s) can be downloaded from the EPO website

(54) **COMPOSITION FOR IN VIVO DELIVERY OF RNA AND PREPARATION METHOD THEREFOR**

(57) The present disclosure relates to a mRNA delivery composition comprising cationic lipid-based liposome. The mRNA delivery composition according to the present disclosure is superb in terms of storage stability and exhibits high intracellular delivery and expression rates *in vivo* and thus, can enhance the stability and efficiency of mRNA vaccines for cancer therapy or mRNA vaccines for prevention of viral infections.

Figure 5

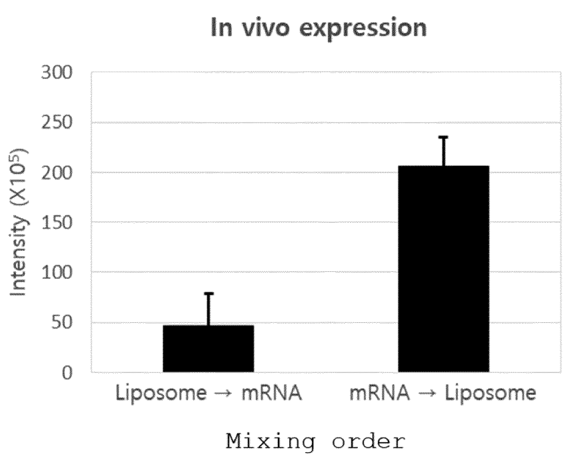


EP 4 306 132 A1

## Description

Technical Field

The present disclosure relates to a composition for *in vivo* delivery of mRNA and, more specifically, to a composition for *in vivo* delivery of mRNA comprising cationic lipid-based liposome and a preparation method therefor.
This application claims priority to Korean Patent Application No. 10-2021-0029929 filed in the Korean Intellectual Property Office on 8 March 2021, the disclosure of which is incorporated herein by reference.

Background Art

Gene therapy and genetic vaccines are technologies that were already proven and generally applied in the medical field, and targets thereof may be not only genetic diseases but also autoimmune diseases, infectious diseases, cancer- or tumor-related diseases, and inflammatory diseases.
Genetic vaccines have been developed since it was reported that when DNA and RNA encoding a target gene were directly injected into animals, the target gene was expressed in living animals and this expression enables immunization (Wolff JA et al. Science, 247:1465-8, 1990).
Genetic vaccination allows evoking a desired immune response to characteristic components of bacterial surfaces, viral particles, and selected antigens such as tumor antigens. Generally, vaccination is one of the pivotal achievements of modern medicine. However, effective vaccines are currently available only for a limited number of diseases. Accordingly, infections that are not preventable by vaccination still affect millions of people every year.
DNA and RNA may be used as nucleic acid molecules for administration in the context of gene therapy or genetic vaccination, and DNA is known to be relatively stable and easy to handle compared with RNA.
However, the use of DNA bears the risk of undesired insertion of the administered DNA-fragments into the patient's genome potentially resulting in loss of function of the impaired genes. As a further risk, the undesired generation of anti-DNA antibodies has emerged, and another drawback is the limited expression level of the encoded peptide or protein that is achievable upon DNA administration and its transcription/translation. The expression level of the administered DNA is dependent on the presence of specific transcription factors, which regulate DNA transcription, and in the absence of the specific transcription factors, DNA transcription will not yield satisfying amounts of RNA, and as a result, the level of translated peptide or protein obtained is also limited.
On the other hand, when RNA is used for gene administration, RNA does not require transcription, and thus the protein may be synthesized directly in the cytoplasm without the need to enter the nucleus, like DNA, so there is no fear of causing unwanted genetic damage due to insertion into cell chromosomes. Moreover, RNA, having a shorter half-life than DNA, does not induce long-term genetic modification (Sayour EJ et al., J Immunother Cancer 2015;3:13, 2015). When a general RNA vaccine is delivered into cells, the RNA vaccine is activated in a short time to thus express a target protein, and is destroyed by an enzymatic reaction within a few days, and the specific immune response to the expressed target antigen (protein) remains.
In addition, when RNA is used for gene administration, RNA acts by passing only through the cell membrane without the need to pass through the nuclear membrane, so RNA is capable of expressing the same amount of target protein as DNA even when used in a smaller amount than DNA. Additionally, RNA itself has immunopotentiation, and thus can exhibit the same immune effect even when administered in a smaller amount than DNA.
By using RNA instead of DNA for genetic vaccination, the risk of undesired genomic integration and generation of anti-DNA antibodies is minimized or avoided. However, RNA is considered to be a rather unstable molecular species which may readily be degraded by ubiquitous RNAses.
Despite a lot of progress made in the last years, there is still a need in the art for providing a method in which the administration is not severely impaired by early degradation of the antigen or by an inefficient translation of the mRNA due to inefficient release of the mRNA in the cell. Furthermore, there is an urgent need to decrease the dose of mRNA vaccines to decrease potential safety concerns and to make the vaccines affordable for the third world. Nucleic acid-based therapeutics, such as vaccines, have enormous potential, but there remains a need for more effective delivery of nucleic acids to appropriate sites within a cell or organism in order to realize this potential.
However, two problems currently face the use of oligonucleotides in the purposes of treatment and prevention. First, free RNAs are susceptible to nuclease digestion in plasma. Second, free RNAs have limited ability to gain access to the intracellular compartment where the relevant translation machinery resides. Lipid nanoparticles formed from cationic lipids with other lipid components, such as neutral lipids, cholesterol, PEG, PEGylated lipids, and nucleic acids, have been used to block degradation of the RNAs in plasma and facilitate the cellular uptake of the oligonucleotides.
In the use of lipid-based delivery system, such as liposome or lipid nanoparticle, a method in which mRNA is usually adsorbed to the outside or encapsulated inside is used. Especially, mRNA, when adsorbed to the outside, is known to be generally present as not a single liposome but an aggregate of a liposome and a nucleic acid, and the

adsorption strength varies and the stability is influenced depending on the combination of lipids, the state of nucleic acids, and the ratio of nucleic acids and liposome, the optimization of which is thus needed.

In terms of the expression ability of mRNA, even if the *in vitro* expression of a delivery system is verified, the results of *in vivo* expression may be different from those of *in vitro* expression. *In vivo,* there may be many factors that reduce the efficiency of delivery into cells, such as the reduction of half-life and bio-distribution due to the aggregation with plasma proteins, a difference in the cellular uptake manner depending on the lipid configuration, and the barrier by extracellular matrix (ECM), according to the lipid configuration and physical and chemical properties of delivery systems. Accordingly, the optimization of delivery systems is absolutely needed in the *in vivo* delivery, unlike *in vitro* delivery.

Disclosure of Invention

Technical Problem

The present inventors have made intensive research efforts to find techniques for improving a delivery system capable of inducing the stable protein expression by stably *in vivo* delivering mRNA to increase the intracellular expression efficiency. As a result, the present inventors prepared a cationic liposome-based "liposome + mRNA complex" through a specific process and established that the "liposome + mRNA complex" prepared by the corresponding process can show high intracellular delivery rates and expression rates *in vivo,* and thus completed the present disclosure.

Accordingly, an aspect of the present disclosure is to provide a composition for mRNA delivery comprising cationic lipid-based liposome having high *in vivo* delivery rates and expression rates.

Another aspect of the present disclosure is to provide a pharmaceutical composition comprising the composition for mRNA delivery as an active ingredient for prevention or treatment of a disease selected from the group consisting of cancer, tumors, autoimmune diseases, genetic diseases, inflammatory diseases, viral infections, and bacterial infections.

Still another aspect of the present disclosure is to provide a vaccine comprising the composition for mRNA delivery as an active ingredient.

Still another aspect of the present disclosure is to provide a functional cosmetic composition comprising the composition for mRNA delivery as an active ingredient.

Still another aspect of the present disclosure is to provide a method for preparing a composition for mRNA delivery.

Solution to Problem

The present inventors have made intensive research efforts to find techniques for improving a delivery system capable of inducing the stable protein expression by stably *in vivo* delivering mRNA to increase the intracellular expression efficiency. As a result, the present inventors prepared a cationic liposome-based "liposome + mRNA complex" through a specific process and established that the "liposome + mRNA complex" prepared by the corresponding process can show high intracellular delivery rates and expression rates *in vivo.*

The present disclosure is directed to a composition for mRNA delivery comprising cationic lipid-based liposome, a pharmaceutical composition comprising the composition for mRNA delivery as an active ingredient for prevention or treatment of a disease selected from the group consisting of cancer, viral infections, and bacterial infections, a vaccine comprising the composition for mRNA delivery as an active ingredient, a functional cosmetic composition comprising the composition for mRNA delivery as an active ingredient, a method for preparing the composition for mRNA delivery.

Unless defined otherwise, all the technical and scientific terms used herein have the same meanings as commonly understood by one of ordinary skill in the art to which this invention belongs. Generally, the nomenclature used herein is well known and commonly employed in the art.

In the present disclosure, with respect to the vivo gene delivery using nucleic acids, methods for preventing or treating a disease by evoking a desired immune response to characteristic components of bacterial surfaces, viral particles, and selected antigens such as tumor antigens and inducing the expression of therapeutic proteins are developed, and therefore, methods are attempted to be found whereby the intracellular mRNA expression efficiency is increased to exhibit stable effects.

Hence, a composition for mRNA delivery using cationic lipid-based liposome was prepared, and the composition for mRNA delivery was confirmed to exhibit high intracellular delivery rates and expression rates *in vivo.*

In the composition for mRNA delivery using cationic lipid-based liposome of the present disclosure, mRNA may be present in the form of forming a complex together with cationic lipid-based liposome, and thus the composition for mRNA delivery using cationic lipid-based liposome is used in the same meaning as the "liposome + mRNA complex".

Hereinafter, the present disclosure will be described in more detail.

According to an aspect of the present disclosure, there is provided a composition for mRNA delivery comprising

cationic lipid-based liposome.

Herein, the "cationic lipid" includes a lipid having a cationic property continuously without the influence of pH change or an ionic lipid that is converted to a cationic form by pH change.

The cationic lipid may be 1,2-dioleoyl-3-trimethylammonium-propane (DOTAP), dimethyl dioctadecyl ammonium bromide (DDA), 3β-[N-(N',N'-dimethyl aminoethane)-carbamoyl-cholesterol (DC-Chol), 1,2-dioleoyloxy-3-dimethylammonium-propane (DODAP), 1,2-di-O-octadecenyl-3-trimethylammonium-propane (DOTMA), 1,2-dimyristoleoyl-sn-glycero-3-ethylphosphocholine (14:1 Etyle PC), 1-palmitoyl-2-oleoyl-sn-glycero-3-ethylphosphocholine (16:0-18:1 Ethyl PC), 1,2-dioleoyl-sn-glycero-3-ethylphosphocholine (18:1 Ethyl PC), 1,2-distearoyl-sn-glycero-3-ethylphosphocholin (18:0 Ethyl PC), 1,2-dipalmitoyl-sn-glycero-3-ethylphosphocholine (16:0 Ethyl PC), 1,2-dimyristoyl-sn-glycero-3-ethylphosphocholine (14:0 Ethyl PC), 1,2-dilauroyl-sn-glycero-3-ethylphosphocholin (12:0 Ethyl PC), N1-[2-((1S)-1-[(3-aminopropyl)amino]-4-[di(3-aminopropyl)amino]butylcarboxamido)ethyl]-3,4-di[oleyloxy]-benzamide (MVL5), 1,2-dimyristoyl-3-dimethylammonium-propane (14:0 DAP), 1,2-dipalmitoyl-3-dimethylammonium-propane (16:0 DAP), 1,2-distearoyl-3-dimethylammonium-propane (18:0 DAP), N-(4-carboxybenzyl)-N,Ndimethyl-2,3-bis(oleoyloxy)propan-1-aminium (DOBAQ), 1,2-stearoyl-3-trimethylammonium-propane (18:0 TAP), 1,2-dipalmitoyl-3-trimethylammonium-propane (16:0 TA), 1,2-dimyristoyl-3-trimethylammonium-propane (14:0 TAP), and/or N4-cholesteryl-spermine (GL67), and preferably 1,2-dioleoyl-3-trimethylammonium propane (DOTAP) or C12-200, but is not limited thereto.

Specifically, the "2-dioleoyl-3-trimethylammonium propane (DOTAP)", which is a cationic emulsifier having a structure of Chemical Formula 1 below, is used as a fabric softener and recently as a nucleic acid carrier that forms liposome.

[Chemical Formula 1]

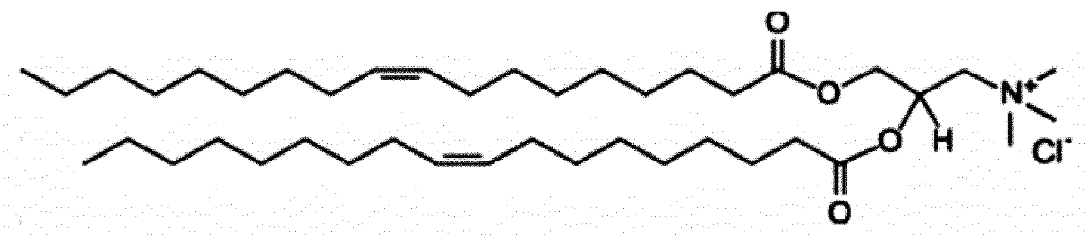

The composition for mRNA delivery comprising the cationic lipid-based liposome of the present disclosure may further comprise a neutral lipid.

Herein, the "neutral lipid" includes a lipid having neutral property continuously without the influence of pH change or an ionic lipid that is converted to a neutral form by pH change.

The neutral lipid may be 1,2-dioleoyl-sn-glycero-3-phosphoethanolamine (DOPE), 1,2-dimyristoyl-sn-glycero-3-phosphorylcholine (DMPC), 1,2-dioleoyl-sn-glycero-3-phosphocholine (DOPC), 1,2-dipalmitoyl-sn-glycero-3-phosphocholine (DPPC), 1,2-distearoyl-sn-glycero-3-phosphocholine (DSPC), 1,2-dilinoleoyl-sn-glycero-3-phosphocholine (DLPC), phosphatidylserine (PS), phosphoethanolamine (PE), phosphatidylglycerol (PG), phosphoric acid (PA), and/or phosphatidylcholine (PC), and preferably, 1,2-dioleoyl-sn-glycero-3-phosphoethanolamine (DOPE), but is not limited thereto.

Specifically, the "1,2-Dioleoyl-sn-glycero-3-phosphoethanolamine (DOPE)" has a structure of Chemical Formula 2 below and is used as an auxiliary lipid for a cationic liposome.

[Chemical Formula 2]

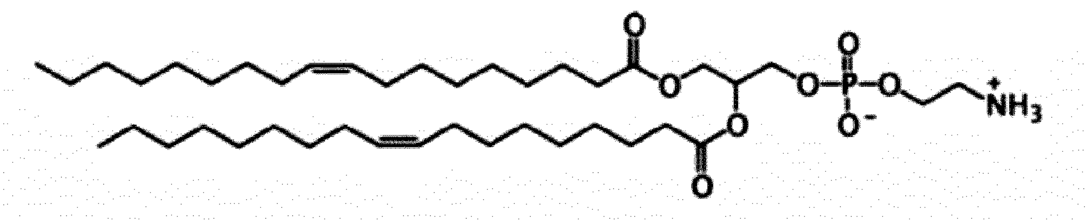

In the present disclosure, the weight ratio of the cationic lipid and the neutral lipid may be 1:9 - 9.5:0.5, 2:8 - 9:1, 3:7 - 8:2, or 4:6 - 7:3, but is not limited thereto.

The weight ratio, when out of the above ranges, may significantly reduce the efficiency of mRNA delivery.

In addition, the composition for mRNA delivery comprising the cationic lipid-based liposome of the present disclosure may further comprise cholesterol.

In the present disclosure, the weight ratio of the cationic lipid and the cholesterol may be 6:1 - 1:3, 4:1 - 1:2.5, 3:1 - 1:2, or 2:1 - 1:1.5, but is not limited thereto.

When the cationic lipid, neutral lipid, and cholesterol are all contained in the liposome according to the present disclosure, the weight ratio of the cationic lipid, neutral lipid, and cholesterol may be 1-9.5:9-0.5:0.05-3, 3-8:7-1:0.45-7.0, or 1-3.5:1-3.5:0.5-3, but is not limited thereto.

The weight ratio, when out of the above ranges, may significantly reduce the efficiency of mRNA delivery.

For example, when further comprising cholesterol, liposome may be prepared by mixing cholesterol with DOTAP:DOPE=1:1 at a weight ratio of 0.2 - 0.85 or 0.5 - 0.85.

Furthermore, the composition for mRNA delivery comprising the cationic lipid-based liposome of the present disclosure may further comprise one or more factors for delivery, such as protamine, albumin, transferrin, protein transduction domains (PTD), cell penetrating peptide (CPP), and macrophage targeting moiety.

Furthermore, the composition for mRNA delivery comprising the cationic lipid-based liposome of the present disclosure may further comprise an immune booster.

The immune booster may be a substance group, which corresponds to a pathogen-associated molecular pattern (PAMP) to respond to pathogen recognition receptors (PRRs), detoxified lipooligosaccharide (dLOS), CpG DNA, lipoprotein, flagella, poly I:C, saponins, squalene, tricaprin, and/or 3D-MPL, but is not limited thereto.

Specifically, the detoxified lipooligosaccharide (dLOS) may be a substance disclosed in Korean Patent Nos. 1509456 and 2042993, but is not limited thereto.

In the composition for mRNA delivery of the present disclosure, the mixing ratio of a mRNA delivery system represented as liposome and mRNA may be expressed by N:P ratio, and the N:P ratio may influence the expression and the stability of the delivery system.

The N:P ratio of liposome and mRNA may be 0.23 - 1.39:1, 0.3 - 1.3:1, 0.4 - 1.2:1, 0.5 - 1.1:1, 0.6 - 1.0:1, 0.6 - 0.9:1, 0.6 - 0.8:1, or 0.7:1, but is not limited thereto.

In the composition for mRNA delivery of the present disclosure, the mRNA may encode a peptide or protein that may act as an immunogen.

The mRNA may be an mRNA having at least one open reading frame (ORF) that can be translated by a cell or an organism provided with that mRNA. The product of this translation is a peptide or protein that may act as an antigen, preferably as an immunogen. The product may also be a fusion protein composed of two or more immunogens, for example, a fusion protein that consists of two or more epitopes, peptides, or proteins derived from the same or different viral proteins, wherein the epitopes, peptides, or proteins may be linked by linker sequences.

In addition, the mRNA may be understood to be an artificial mRNA, that is, a non-naturally occurring mRNA molecule. The artificial mRNA molecule may be understood as a non-natural mRNA molecule. Such a mRNA molecule may be non-natural due to its individual sequence (which does not occur naturally) and/or due to other modifications, e.g. structural modifications of nucleotides which do not occur naturally. The artificial mRNA molecule may be designed and/or generated by genetic engineering methods corresponding to a desired artificial sequence of nucleotides (heterologous sequence).

Furthermore, the mRNA may exhibit a modification increasing resistance to *in vivo* degradation (e.g., degradation by an exo- or endo-nuclease) and/or ex vivo degradation (e.g., by the manufacturing process prior to vaccine administration, e.g., in the course of the preparation of the vaccine solution to be administered). The stabilization of RNA can be achieved by providing, for example, a 5'-CAP-Structure, a Poly-A-Tail, or any other UTR-modification. It can also be achieved by chemical modification or modification of the G/C-content of the nucleic acid. Various other methods are known in the art and conceivable in the context of the invention.

In accordance with another aspect of the present disclosure, there is provided a pharmaceutical composition, comprising the above-described composition for mRNA delivery as an active ingredient, for prevention or treatment of a disease selected from the group consisting of cancer, tumors, autoimmune diseases, genetic diseases, inflammatory diseases, viral infections, and bacterial infections.

As used herein, the term "prevention" refers to any action that inhibits the above-described diseases or delays the progression thereof by administration of the pharmaceutical composition according to the present disclosure.

As used herein, the term "treatment" refers to any action to alleviates or advantageously changes symptoms of the above-described diseases by administration of the pharmaceutical composition according to the present disclosure.

The pharmaceutical composition of the present disclosure may comprise at least one pharmaceutically or physiologically acceptable carrier, diluent, or excipient in combination. The pharmaceutical composition may contain: a buffer, such as neutral buffered saline, phosphate buffered saline, or citric acid buffered solution; a carbohydrate, such as glucose, mannose, sucrose or dextran, or mannitol; a protein; a polypeptide or an amino acid such as glycine; an antioxidant; a chelating agent, such as EDTA or glutathione; an adjuvant (e.g., aluminum hydroxide); and a preservative.

The pharmaceutical composition of the present disclosure may be administered orally or parenterally, for example, by intravenous administration, subcutaneous administration, intradermal administration, intramuscular administration, intraperitoneal administration, intratumoral injection, intracerebral administration, intracranial administration, intrapulmonary administration, and rectal administration, but is not limited thereto.

The pharmaceutical composition of the present disclosure is administered at a pharmaceutically effective amount. As used herein, the term "pharmaceutically effective amount" refers to an amount sufficient to treat a disease at a reasonable benefit/risk ratio applicable to medical treatment, and the effective amount may be determined according to the factors including the type and severity of a disease of a patient, activity of a drug, sensitivity to a drug, time of administration, route of administration, and rate of excretion, duration of treatment, and drugs used in combination, and other factors that are well known in the field of medicine.

The pharmaceutical composition of the present disclosure may be administered as an individual medicine or administered in combination with other medicines, and may be administered sequentially or simultaneously with a conventional medicine, and may be administered in either single or multiple doses. It is important to administer an amount at which a maximum effect can be obtained with a minimum amount without side effects considering all of the above factors, and the amount can be easily determined by those skilled in the art.

Specifically, the effective amount of the pharmaceutical composition of the present disclosure may vary depending on the age, sex, condition, and weight of a patient, the absorption rate, inactivity rate, and excretion rate of an active ingredient in the body, the disease type, and the drug used in combination.

Since the pharmaceutical composition of the present disclosure comprises the above-described composition for mRNA delivery as an active ingredient, descriptions of overlapping contents are omitted to avoid excessive complexity in the present specification.

In accordance with still another aspect of the present disclosure, there is provided a method for preventing or treating cancer, tumors, autoimmune diseases, inflammatory diseases, viral infections, and bacterial infections, the method including administering the composition for mRNA delivery to a patient in need of prevention or treatment of cancer, tumors, autoimmune diseases, inflammatory diseases, viral infections, and bacterial infections.

The dosage of mRNA may be, per dose, 1 - 5 μg, 5 - 10 μg, 10 - 15 μg, 15 - 20 μg, 10 - 25 μg, 20 - 25 μg, 20 - 50 μg, 30 - 50 μg, 40 - 50 μg, 40 - 60 μg, 60 - 80 μg, 60 - 100 μg, 50 - 100 μg, 80 - 120 μg, 40 - 120 μg, 40 - 150 μg, 50 - 150 μg, 50 - 200 μg, 80 - 200 μg, 100 - 200 μg, 120 - 250 μg, 150 - 250 μg, 180 - 280 μg, 200 - 300 μg, 50 - 300 μg, 80 - 300 μg, 100 - 300 μg, 40 - 300 μg, 50 - 350 μg, 100 - 350 μg, 200 - 350 μg, 300 - 350 μg, 320 - 400 μg, 40 - 380 μg, 40 - 100 μg, 100 - 400 μg, 200 - 400 pg, or 300 - 400 μg, but is not limited there-.

The vaccine may be a vaccine against viruses that can infect humans and animals, such as influenza, corona virus, herpes zoster, human papilloma virus, Zika virus, herpes virus, AIDS virus, SFTS virus, measles virus, chickenpox virus, Ebola virus, MERS virus, hepatitis virus, avian influenza, rabies virus, and foot-and-mouth disease virus, but is not limited thereto.

The vaccine comprises at least one mRNA having an open reading frame encoding a polypeptide of at least one viral antigen or an immunogenic fragment thereof.

Since the treatment method of the present disclosure includes the above-described pharmaceutical composition as an active ingredient, descriptions of overlapping contents are omitted to avoid excessive complexity in the present specification.

In accordance with still another aspect of the present disclosure, there is provided a vaccine comprising the above-described composition for mRNA delivery as an active ingredient.

The vaccine may comprise the above-described composition for mRNA delivery at an appropriate concentration in consideration of the weight, age, dietary stage, and/or immunity of a subject to be administered, within the range of the purpose of preventing diseases caused by peptides or proteins in which the above-described mRNA may act as an immunogen.

The vaccine may further comprise at least one selected from the group consisting of a carrier, a diluent, an excipient, and an adjuvant. The carrier is not particularly limited to its type, but may include any all solvents, dispersion media, coatings, stabilizers, preservatives, antibacterial and antifungal agents, isotonic agents, absorption delaying agents, and the like.

The vaccine may be administered by oral, parenteral, subcutaneous, intramuscular, intradermal, sublingual, dermal, intrarectal, transmucosal, surface through inhalation, or buccal administration, or a combination thereof.

Depending on the desired period and effectiveness of vaccination or treatment, the vaccine may be administered once or several times, and also intermittently, for example, in the same dosage or different dosages daily for several days, several weeks or several months. An injection may be injected at a desired amount or be injected by subcutaneous or nasal spray, or alternatively, may be continuously injected.

Since the vaccine of the present disclosure comprises the above-described composition for mRNA delivery as an active ingredient, descriptions of overlapping contents are omitted to avoid excessive complexity in the present specification.

In accordance with still another aspect of the present disclosure, there is provided a functional cosmetic composition comprising the above-described composition for mRNA delivery as an active ingredient.

The cosmetic composition according to the present disclosure may comprise ingredients that are ordinarily used in cosmetic compositions, and may contain, for example, ordinary adjuvants, such as a metal ion sequestering agent, active components (e.g., sodium hyaluronate and tocopheryl acetate, etc.), a preservative, a thickener, and a fragrance, and a carrier.

In addition, the cosmetic composition according to the present disclosure may be prepared in the form of a general formulation in the art, for example, an emulsified formulation or a solubilized formulation. Examples of the emulsified formulation may be a nourishing lotion, a cream, an essence, and the like, and examples of the solubilizer formulation may be a softening lotion and the like. In addition, the cosmetic composition of the present disclosure may be prepared in the form of not only a cosmetic product, but also an adjuvant that can be topically or systemically applied and is ordinarily used in a field of dermatology, by comprising a dermatologically acceptable medium or base.

Examples of an appropriate formulation for the cosmetic product may be a solution, a gel, a solid or kneaded anhydrous product, an emulsion obtained by dispersing an oil in water, a suspension, a microemulsion, a microcapsule, micro-granules, an ionic (liposome) or non-ionic vesicle dispersant, a cream, a toner, a lotion, a powder, an ointment, a spray, or a concealer stick. The cosmetic composition may be prepared in the form of a foam, or in the form of an aerosol composition further comprising a compressed propellant.

The cosmetic composition of the present disclosure may further comprise an adjuvant that is commonly used in the cosmetology or dermatology, for example, a fatty substance, an organic solvent, a solubilizing agent, a thickening agent, a gelling agent, a softening agent, an antioxidant, a suspending agent, a stabilizer, a foaming agent, an aroma, a surfactant, water, an ionic or nonionic emulsifier, a filler, a metal sequestering agent, a chelating agent, a preservative, vitamins, a blocking agent, a wetting agent, an essential oil, a dye, a pigment, a hydrophilic or lipophilic activating agent, lipid vesicles, or any other component used in a cosmetic product. In addition, the above ingredients may be introduced at amounts that are generally used in the field of dermatology.

Examples of a product to which the cosmetic composition of the present disclosure may be added include cosmetic products, such as an astringent lotion, a softening lotion, a nourishing lotion, various creams, an essence, a pack, and a foundation, as well as cleansings, facial cleansers, soaps, treatments, and beauty essences.

Specific formulations of the cosmetic composition of the present disclosure include a skin lotion, a skin softener, a skin toner, an astringent, a lotion, a milk lotion, a moisture lotion, a nourishing lotion, a massage cream, a nourishing cream, a moisture cream, a hand cream, an essence, a nourishing essence, a pack, a soap, a shampoo, a cleansing foam, a cleansing lotion, a cleansing cream, a body lotion, a body cleanser, a milky lotion, a pressed powder, a loose powder, a patch, a sprayer, and the like.

Since the cosmetic composition of the present disclosure comprises the above-described composition for mRNA delivery as an active ingredient, descriptions of overlapping contents are omitted to avoid excessive complexity in the present specification.

In accordance with still another aspect of the present disclosure, there is provided a method for preparing a composition for mRNA delivery, the method including mixing mRNA and liposome.

The mRNA and/or liposome may be provided in the form of a lyophilized powder or in the form of being dissolved in an appropriate solution or buffer. The mRNA and/or liposome, when provided in a lyophilized form, may be used after being dissolved in an appropriate solution or buffer.

In a specific example of the present disclosure, the present inventors characterized mRNA-liposome complexes prepared by adjusting the mixing order of liposome and mRNA and confirmed the *in vivo* mRNA expression according to the mixing order of liposome and mRNA.

In the present disclosure, the mRNA and liposome may be mixed by adding in the order of mRNA → liposome.

By mixing mRNA and liposome in the above order, the *in vivo* delivery and expression rates of the prepared mRNA-liposome complexes can be increased.

The method for preparing a composition for mRNA delivery of the present disclosure may further include mixing an immune booster.

The immune booster may be provided in the form of a lyophilized powder or in the form of being dissolved in an appropriate solution or buffer. The immune booster, when provided in a lyophilized form, may be used after being dissolved in an appropriate solution or buffer.

In a specific example of the present disclosure, the present inventors confirmed the *in vivo* mRNA expression depending on the mixing order of liposome, mRNA, and an immune booster with respect to mRNA-liposome complexes prepared by controlling the mixing order of liposome, mRNA, and immune booster.

In the present disclosure, the mRNA, liposome, and immune booster may be mixed by adding in the order of the immune booster- mRNA → liposome.

By mixing the mRNA, liposome, and immune booster in the above order, the *in vivo* delivery and expression rates of the prepared mRNA-liposome complexes can be increased.

Since the mRNA and liposome of the present disclosure are active ingredients of the above-described composition for mRNA delivery, descriptions of overlapping contents are omitted to avoid excessive complexity in the present specification.

Advantageous Effects of Invention

The composition for mRNA delivery comprising cationic liposome according to the present disclosure has excellent storage stability and shows high intracellular delivery and expression rates *in vivo,* and thus can improve the stability and efficiency of mRNA vaccines for cancer treatment/prevention or mRNA vaccines for preventing viral or bacterial infections.

Brief Description of Drawings

FIG. 1 shows the results of mRNA expression, in mice, of liposome-mRNA complexes prepared with different amounts of mRNA used.

FIG. 2 shows the results of mRNA expression, in mice, of liposome-mRNA complexes using liposome, prepared with different ratios of DOTAP:DOPE.

FIG. 3 shows the results of mRNA expression, in mice, of liposome-mRNA complexes using liposome, prepared with different ratios of DOTAP:DOPE:cholesterol.

FIG. 4 shows the analysis results of size, dispersity, and zeta potential of samples of mRNA-liposome complexes prepared by different mixing orders of mRNA and liposome.

FIG. 5 shows the results of *in vivo* mRNA expression difference according to the mixing order of mRNA and liposome.

FIG. 6 shows the results of cellular immune response (FIG. 6A), antibody immune response (FIG. 6B), and neutralizing antibody titers (FIG. 6C) after mice were immunized with mRNA-liposome complexes prepared by different mixing orders of mRNA and liposome.

FIG. 7 shows the results of mRNA expression, in mice, of liposome-mRNA complexes prepared with different amounts of dLOS used.

FIG. 8 shows the results of mRNA expression when mice were injected with mRNA-liposome-dLOS complexes prepared by different mixing orders of mRNA, liposome, and dLOS (LP: liposome; R: mRNA; dL: dLOS).

FIG. 9 shows the measurement results of size (FIG. 9A), dispersity (FIG. 9B), and zeta potential (FIG. 9C) according to storage period to determine the stability of mRNA-liposome complexes prepared by different N/P ratios.

Mode for Carrying out the Invention

Hereinafter, the present disclosure will be described in more detail with reference to exemplary embodiments. These exemplary embodiments are only for specifically illustrating the present disclosure, and it would be obvious to those skilled in the art that the scope of the present disclosure is not construed as being limited to the exemplary embodiments.

**Preparation Example 1: Preparation of mRNA-liposome complex**

**Liposome preparation (Film method)**

Chloroform was mixed with DOTAP (Merck & Cie/CH2900014), DOPE (Avanti Polar Lipid) and/or cholesterol (Avanti Polar Lipid), separately, and then completely dissolved at 37°C for 10 minutes to prepare liquid solutions.

The liquid solutions were mixed at a predetermined weight ratio in a round-bottom flask to make a lipid mixture, and chloroform was blown off by volatilization in a rotary evaporator (Buchi/B491_R200) at 60°C for 30 minutes to manufacture a lipid membrane film on the wall surface of the flask.

A 20 mM HEPES buffer (pH 7.4) comprising 4% (w/v) sucrose was placed in the flask in which the lipid membrane film was manufactured, and the lipid membrane was dissolved at 60°C to form liposome with a liposome concentration of 7.5 mg/mL. The size, zeta potential, and dispersity of the formed liposome were measured by a dynamic light scattering analyzer. The remaining liposome were stored at 4°C before tests.

**mRNA-liposome complex preparation**

The prepared liposome (LP-DOTAP/DOPE/Chol (40:40:20, w/w/w) or LP-DOTAP/DOPE (50:50, w/w)) and Renilla luciferase mRNA (SEQ ID NO: 1) were mixed with a 20 mM HEPES buffer (pH 7.4) comprising 4% sucrose to

prepare mRNA-liposome complexes.

Particularly, the 7.5 mg/mL liposome solution (LP-DOTAP/DOPE/Chol (40:40:20, w/w/w)) employed a sample refrigerated immediately after the preparation or within up to 2 weeks after the preparation. The 1 mg/mL Renilla luciferase mRNA (SEQ ID NO: 1) solution was stored at -70°C, and dissolved on ice immediately before use. The 20 mM HEPES buffer (pH 7.4) comprising 4% sucrose was prepared immediately before tests, or prepared on the day before tests and then refrigerated.

The N/P ratio, which is the mixing ratio of liposome and mRNA, was calculated by the following calculation formula.

$$\text{N/P ratio} = \frac{\text{DOTAP mass (g)}}{DOTAP\ molecular\ weight} : \frac{\text{mRNA mass (g)}}{mRNA\ molecular\ weight} \times mRNA\ base\ pair$$

**Example 1: mRNA expression according to mRNA content of mRNA-liposome complex**

In the preparation of the liposome-mRNA complexes in Preparation Example 1, the complexes (N/P ratio=0.7) were prepared while the amount of mRNA was varied to 5 μg, 10 μg, and 20 μg and for each situation, the amounts of liposome of DOTAP:DOPE:Chol (40:40:20, w/w/w) were 18.75 μg, 37.5 μg, and 75 μg. Thereafter, the *in vivo* expression levels thereof were examined.

Specifically, 100 μL of each of the liposome-mRNA complexes was intramuscularly (I.M.) injected into the thigh deltoid muscle of shaved mice (7-week-old C57BL/6N female, Orient Bio, Central Experimental Animal).

Six hours after administration, the mice were anesthetized with avertin (250 mg/kg) and then intravenously (I.V.) injected with 200 μL of a 0.15 μg/μL substrate, which was obtained by adding 2.4 mL of 1x PBS to the ViviRen™ *in vivo* Renilla luciferase substrate stock solution.

Immediately after administration, the mice were positioned in an IVIS system (Ami-HTX, USA) and then photographed (Xenogen IVIS-200) while the exposure time was set to 60 seconds, and the expression level (region of interest, ROI) of luciferase at the site of administration was numerically quantified using Aura Imaging Software (Spectral Instruments Imaging, USA).

As a result, as can be confirmed in FIG. 1, there was no difference in expression level for 5 μg of mRNA and 10 μg of mRNA, but 20 μg of mRNA showed increases of 135% and 170% compared with 5 μg of mRNA and 10 μg of mRNA, respectively. The *in vivo* expression level was increased at a certain concentration or more of mRNA.

**Preparation Example 2: Preparation of mRNA-liposome complexes with adjusted mixing ratios of cationic lipid:neutral lipid:cholesterol (DOTAP:DOPE:cholesterol) in liposome**

**2-1. Adjustment of mixing ratio of DOTAP:DOPE**

Liposome was prepared at DOTAP:DOPE ratios (w/w) shown in Table 1 below and then mixed with mRNA to prepare liposome-mRNA complexes. Liposome comprising 30 μg of DOTAP with respect to 20 μg of mRNA (Renilla luciferase mRNA, SEQ ID NO: 1) were used so that the NP ratio (molar ratio) of the liposome-mRNA complexes was 0.7:1. Particularly, LNP (C12-200:DSPC:Chol:DMG-PEG2000 = 50:10:38.5:1.5) obtained by mixing C12-200, DSPC, cholesterol, and DMG-PEG2000 at a ratio of 50:10:38.5:1.5 by NanoAssemblr Ignite (Precision NanoSystems) using a protocol recommended by the manufacturer was used as a liposome control.

Table 1

| Test group | 1 | 2 | 3 | 4 | 5 | 6 | 7 |
|---|---|---|---|---|---|---|---|
| DOTAP | 20 | 30 | 40 | 50 | 70 | 90 | 100 |
| DOPE | 80 | 70 | 60 | 50 | 30 | 10 | 0 |

**2-2. Adjustment of mixing ratio of DOTAP:DOPE:cholesterol**

Liposome was prepared at DOTAP:DOPE:cholestrol ratios (w/w) shown in Table 2 below and then mixed with mRNA to prepare liposome-mRNA complexes. Liposome comprising 30 μg of DOTAP with respect to 20 μg of mRNA (Renilla luciferase mRNA, SEQ ID NO: 1) were used so that the NP ratio of the liposome-mRNA complexes was 0.7:1. Particularly, invivofectamine (Thermo Fisher Scientific) was used as a liposome control.

Table 2

| Test group | 1 | 2 | 3 | 4 | 5 | 6 | 7 | 8 | 9 |
|---|---|---|---|---|---|---|---|---|---|
| DOTAP | 50 | 45 | 40 | 35 | 30 | 20 | 40 | 60 | 80 |
| DOPE | 50 | 45 | 40 | 35 | 30 | 60 | 40 | 20 | 0 |
| Chol | 0 | 10 | 20 | 30 | 40 | 20 | 20 | 20 | 20 |

## Example 2: mRNA expression according to mixing ratio of cationic lipid:neutral lipid:cholesterol (DOTAP:DOPE:cholesterol) in liposome

### 2-1. In *vivo* expression level according to mixing ratio of DOTAP:DOPE

The *in vivo* expression of each of the liposome-mRNA complexes prepared in Preparation Example 2-1 was examined.

As a result, as can be confirmed in FIG. 2, the liposome-mRNA complexes using liposome prepared at DOTAP:DOPE ratios of 40:60, 50:50, and 70:30 showed higher mRNA expression levels in the mice.

### 2-2. In *vivo* expression level according to mixing ratio of DOTAP:DOPE:cholesterol

The *in vivo* expression of each of the liposome-mRNA complexes prepared in Preparation Example 2-2 was examined by the same method as in Example 1.

As a result, as can be confirmed in FIG. 3, when the DOTAP:DOPE ratio was fixed at 1:1 and the percentage of cholesterol was adjusted, the liposome-mRNA complexes using liposome prepared at DOTAP:DOPE:cholesterol ratios of 40:40:20 and 35:35:30 showed higher mRNA expression levels in the mice. In addition, when the percentage of cholesterol was fixed, the liposome-mRNA complexes using liposome prepared at DOTAP:DOPE:cholesterol ratios of 20:60:20 and 40:40:20 showed higher mRNA expression levels in the mice.

## Example 3: Characterization according to mixing ratio of cationic lipid:neutral lipid:cholesterol (DOTAP:DOPE:cholesterol) in liposome

The liposome and the mRNA-liposome complexes prepared in Preparation Example 2-2 were diluted to 1/10 with 20 mM HEPES buffer (pH 7.4) comprising 4% sucrose. Dynamic light scattering (DLS) analysis was performed using a Zetasizer Nano ZSP (Malvern Pnanlytical) to measure the size, dispersity (PDI), and zeta potential of the complexes.

As a result, as can be confirmed in Table 3 (liposome) and Table 4 (liposome-mRNA complexes) below, the liposome had a size of 100-200 nm and a dispersity of less than 0.4. When DOTAP and DOPE had the same percentage, the size and dispersity of particles were larger as the cholesterol content was higher, and under the same cholesterol content of 20, the size and dispersity of particles tended to be larger as the DOPE content was higher than the DOATP content (Table 3). The liposome-mRNA complexes overall had a size of 180-240 nm and a dispersity of less than 0.3, and these numerical values were greater than those of liposome not mixed with mRNA. When mRNA was mixed, no change in particle size was observed according to the cholesterol content.

Table 3

| LP-DOTAP/DOPE/Chol (Weight ratio, 2 mg/mL) | | | Analysis item | |
|---|---|---|---|---|
| DOTAP | DOPE | Chol | Particle size (d, nm) | Dispersity |
| 50 | 50 | 0 | 116.9 | 0.161 |
| 45 | 45 | 10 | 124.0 | 0.161 |
| 40 | 40 | 20 | 128.3 | 0.162 |
| 35 | 35 | 30 | 130.8 | 0.160 |
| 30 | 30 | 40 | 172.3 | 0.346 |
| 20 | 60 | 20 | 192.3 | 0.225 |

(continued)

| LP-DOTAP/DOPE/Chol (Weight ratio, 2 mg/mL) | | | Analysis item | |
|---|---|---|---|---|
| DOTAP | DOPE | Chol | Particle size (d, nm) | Dispersity |
| 60 | 20 | 20 | 131.8 | 0.163 |
| 80 | 0 | 20 | 111.5 | 0.156 |

Table 4

| LP-DOTAP/DOPE/Chol + mRNA | | | Analysis item | | |
|---|---|---|---|---|---|
| DOTAP | DOPE | Chol | Particle size (d, nm) | Dispersity Zeta | potential (mV) |
| 50 | 50 | 0 | 197.8 | 0.208 | -63.5 |
| 45 | 45 | 10 | 195.8 | 0.188 | -63.9 |
| 40 | 40 | 20 | 222.2 | 0.244 | -61.2 |
| 35 | 35 | 30 | 184.0 | 0.176 | -60.0 |
| 30 | 30 | 40 | 223.0 | 0.258 | -56.0 |
| 20 | 60 | 20 | 236.7 | 0.210 | -48.3 |
| 60 | 20 | 20 | 190.3 | 0.179 | -66.2 |
| 80 | 0 | 20 | 187.6 | 0.207 | -68.0 |

**Preparation Example 3: Preparation of mRNA-liposome complexes with adjusted mixing orders of liposome and mRNA**

The 1 mg/mL Renilla luciferase mRNA solution stored at -70°C and the refrigerated liposome solution (LP-DOTAP/DOPE/Chol (40:40:20, w/w/w)) and 20 mM HEPES buffer (pH 7.4) comprising 4% sucrose in Preparation Example 1 were used.

The following preparation method is an example of the preparation of 400 μL of a liposome-mRNA complex, and the complex was prepared by increasing the weights at a predetermined ratio as necessary, and solution mixing was performed by known methods, such as stirring and pipetting.

**3-1. Order of mRNA → Liposome**

The 340 μL of 20 mM HEPES buffer (pH 7.4) comprising 4% sucrose was taken using a 200P tip only for RNA and dispensed into microtubes. The 40 μL of completely thawed 1 mg/mL Renilla luciferase mRNA solution (40 μg mRNA) was taken using a 200P tip and placed in the microtubes in which the buffer was dispensed, followed by pipetting about 10 times. The 20 μL of 7.5 mg/mL liposome solution (LP-DOTAP/DOPE/Chol (40:40:20, w/w/w) (150 μg liposome)) was taken using a 200P tip and placed in the microtubes in which the buffer and the mRNA solution were dispensed, followed by pipetting about 30 times. Last, the sample in which the buffer, the mRNA solution, and the liposome solution were mixed was further mixed by pipetting about 10 times with a 1000P tip.

**3-2. Order of Liposome → mRNA**

A 340 μL of 20 mM HEPES buffer (pH 7.4) comprising 4% sucrose was taken using a 200P tip only for RNA and dispensed into microtubes. The 20 μL of 7.5 mg/mL liposome solution (LP-DOTAP/DOPE/Chol (40:40:20, w/w/w) (150 μg liposome)) was taken using a 200P tip and placed in the microtubes in which the buffer was dispensed, followed by pipetting about 10 times. The 40 μL of completely thawed 1 mg/mL Renilla luciferase mRNA solution (40 μg mRNA) was taken using a 200P tip and placed in the microtubes in which the buffer and the liposome solution were dispensed, followed by pipetting about 30 times. Last, the sample in which the buffer, the mRNA solution, and the liposome solution were mixed was further mixed by pipetting about 10 times with a 1000P tip.

**Example 4: Characterization according to mixing order of liposome and mRNA**

The liposome-mRNA complexes prepared in Preparation Example 3 was subjected to DLS analysis to deduce the means and standard deviations of the size, dispersity (PDI), and zeta potential of the complexes.

As a result, as can be confirmed in FIG. 4, the liposome-mRNA complexes showed no significant differences in the physical properties.

**Example 5: mRNA expression according to mixing order of liposome and mRNA**

The *in vivo* expression of each of the liposome-mRNA complexes prepared according to the different mixing orders in Preparation Example 3 was analyzed by the same method as in Example 1.

As a result, as can be confirmed in FIG. 5, the mRNA expression was prominently high in the complexes prepared by the mixing order of mRNA → liposome.

**Example 6: Identification of immunogenicity according to mixing ratio of liposome and mRNA**

Liposome-mRNA complexes, prepared by different mixing orders by the same method as in Preparation Example 3 except that SARS-CoV-2 S mRNA (SEQ ID NO: 3) was used instead of Renilla luciferase mRNA, were subjected to the following tests.

First, 6-week-old female mice (B6C3F1/slc, Central Experimental Animals) were administered with the liposome-mRNA complexes, prepared by different mixing orders, into the thigh of the left hind leg through a muscular administration route at 0.1 human dose (HD) twice at intervals of 3 weeks.

**6-1. Cytokines**

Splenocyte restimulation

After the mice were sacrificed by cervical dislocation two weeks after the last administration, the spleen was extracted, pooled, and then transferred to a 24-well plate in which PBS supplemented with a 1% penicillin-streptomycin solution (hereinafter, PBS w/antibiotics) was dispensed. The media used are shown in Tables 5 and 6 below.

Table 5

| Complete media | Amount of addition | Final concentration |
|---|---|---|
| RPMI 1640 medium | 439.9 mL | - |
| FBS (non-heat inactivated) | 50 mL | 10%(v/v) |
| Penicillin-streptomycin solution (100x) (antibiotics) | 5 mL | Penicillin (100 U/μL), Streptomycin (100 ug/uL) |
| HEPES buffer solution (1 M) | 5 mL | 10 mM |
| 0.5 M β-mercaptoethanol | 50 μL | 50 uM |
| Recombinant mouse IL-2 | 12.5 μL | 0.5 (ng/μL) |

Table 6

| Basal media | Amount of addition | Final concentration |
|---|---|---|
| RPMI 1640 medium | 439.9 mL | - |
| FBS (non-heat inactivated) | 50 mL | 10% (v/v) |
| Penicillin-streptomycin solution (100x) (antibiotics) | 5 mL | Penicillin (100 U/μL), Streptomycin (100 μg/μL) |
| HEPES buffer solution (1 M) | 5 mL | 10 mM |

In a clean bench, the spleen tissue was picked up by forceps, washed with PBS w/antibiotics, and then transferred to a 60 mm dish comprising 3 mL of basal media, and the tissue was disrupted using a 40 μm cell strainer to isolate splenocytes. The isolated splenocytes were transferred to a 15 mL tube and then centrifuged at 4°C and 3,000 rpm for 5 minutes to remove the supernatant. The cells were suspended in 3 mL of RBC lysis buffer, allowed to stand at room temperature for 3 minutes, and then centrifuged at 4°C and 3,000 rpm for 5 minutes. The supernatant was removed, and the cells were suspended in 3 mL of PBS w/antibiotics and centrifuged at 4°C and 3,000 rpm for 5 minutes. After the supernatant was removed, the cells were suspended in 10 mL of complete media. The cell suspension was diluted to $2\times10^7$ cells/mL by using complete media, and then dispensed at 100 μL/well into a 96-well cell culture plate.

Separately, a PepMix SARS-CoV-2-S1 peptide pool (JPT) and a SARS-CoV-2-S2 peptide pool (JPT) each were dissolved in 50 μL of DMSO in one vial, and then mixed with complete media to a final concentration of 2.5 ug/mL to prepare a SARS CoV-2 spike peptide stimulant.

To a 96-well comprising the cell suspension, the 40 μg/well stimulant and 60 μL/well complete media were added, followed by incubation under conditions of 37°C and 5% $CO_2$ for 72 hours.

<u>IFN-γ concentration</u>

The culture of the stimulated splenocytes was diluted to 1/5 with a reagent diluent (1% BSA), dispensed into a microplate coated with an anti-mouse IFN-γ capture antibody (Jackson) at 100 μL/well, covered with a sealing film, and allowed to stand at room temperature for 2 hours. Thereafter, the solution of each well was removed by an ELISA washer (Tecan/Hydroflexelisa) and washed three times with a washing buffer.

Streptavidin-HRP in an IFN-γ ELISA kit (Mouse IFN-γ Duoset ELISA, R&D systems) was diluted to 1/40 by using a reagent diluent, and then 100 μL/well of the diluted solution was dispensed into an immunoplate, covered with a sealing film, and allowed to stand at room temperature for 20 minutes, and then the solution of each well was removed using an ELISA washer and washed three times using a washing buffer.

The anti-mouse IFN-γ detection antibody in the kit was diluted to 200 ng/mL by using a reagent diluent, and dispensed into an immunoplate at 100 μL/well, covered with a sealing film, and allowed to stand at room temperature for 1 hour. Thereafter, the solution of each well was removed using an ELISA washer (Tecan/Hydroflexelisa) and washed three times using a washing buffer.

A TMB substrate (KPL sureblue TMB microwell peroxidase substrate, Seracare) solution was dispensed into an immunoplate at 100 μL for each well, and incubated in a dark place at room temperature for 15 minutes, and then the incubation was stopped by dispensing a 1 N $H_2SO_4$ solution into each 100 μL in the immunoplate, and the absorbance was measured at 450 nm using an ELISA reader.

As a result, as can be confirmed in FIG. 6A, the IFN-γ concentration was highest in the complexes prepared by the mixing order of mRNA → liposome.

**6-2. IgG titers**

Two weeks after the last administration, the mice were anesthetized with 250 mg/kg Avertin working solution through intraperitoneal administration, and the whole blood was collected through cardiac blood collection. The collected whole blood was transferred to a microtube, allowed to stand at room temperature for 3 hours, and then centrifuged at 4°C and 15,000 rpm for 10 minutes, and the supernatant was transferred to new microtubes to secure serum and stored at -20°C until analysis.

Next, the SARS-CoV-2 receptor binding domain (RBD) antigen (Mybiosource, USA) was diluted to 1 ug/mL with 1x PBS, dispensed at 100 μL/well into an immunoplate, covered with a sealing film, and allowed to stand at 4°C overnight. The solution of each well was removed with an ELISA washer (Tecan/Hydroflexelisa) and washed five times with a washing buffer (500 μL of Tween 20 was added to 1 L of 1x PBS obtained by diluting 20x PBS with purified water). A reagent diluent (prepared by dissolving 1% BSA and 1 g of BSA in 100 mL of PBS) was dispensed at 200 μL/well into the immunoplate, covered with a sealing film, and allowed to stand in an incubator at 37°C for 1 hour. The solution of each well was removed with an ELISA washer and washed five times with a washing buffer. The reagent diluent was dispensed into the immunoplate at 100 μL/well.

The serum obtained above was diluted to 1:50 using a reagent diluent (1% BSA), and then 100 μL of the diluted serum was dispensed into each well of the first row of immunoplate B to G. The sample in each well was mixed by pipetting several times, and then 100 μL was taken from the first row and transferred into each well of the second row, and through such a manner, the sample was subjected to 1/2 serial dilution to the 12th row on the ELISA plates. Particularly, for the evaluation of test suitability, the hyperserum was diluted to 1:200 using a reagent diluent, and then dispensed at 100 μL for each well into the first row of immunoplate H, followed by 1/2 serial dilution in the same manner as described above.

The immunoplate was covered with a sealing film and incubated in an incubator at 37°C for 2 hours. The solution

of each well was removed with an ELISA washer and washed five times with a washing buffer. A goat anti-mouse IgG antibody (Jackson Laboratory) was diluted to 1:5,000 using a reagent diluent, and then the diluted antibody was dispensed into an immunoplate at 100 μL for each well, which was then covered with a sealing film, followed by incubation at 37°C in an incubator for 1 hour. The solution of each well was removed with an ELISA washer and washed five times with a washing buffer.

The TMB substrate solution equilibrated with room temperature was dispensed into an immunoplate at 100 μL for each well, followed by incubation in a dark place at room temperature for 5 minutes. The reaction was stopped by dispensing 1 N $H_2SO_4$ solution into the immunoplate at 100 μL for each well, and the absorbance was measured at 405 nm using an ELISA reader (Biotek/Epoch).

As a result, as can be confirmed in FIG. 6B, the antibody immune response was highest in the complexes prepared by the mixing order of mRNA → liposome.

**6-3. Analysis of surrogate neutralization (%)**

In 1.5 mL microtubes, a negative control (DMEM medium) or 60 μL of each of the immunized mouse serum samples obtained in Example 6-2 and stored at -20°C was mixed with 60 μL of 1:1000 diluted-HRP conjugated RBD and incubated at 37°C for 30 minutes, and 100 μL was dispensed into a microtiter test strip plate, covered with a sealing film, and incubated at 37°C for 15 minutes. The solution of each well was removed with an ELISA washer and washed four times with a 1x washing buffer. The TMB solution was dispensed at 100 μL/well, covered with a sealing film, and incubated in a dark place at room temperature for 15 minutes. Then, the incubation was stopped by dispensing a stop solution at 50 μL/well, and then the optical density was measured at 450 nm using an ELISA reader.

As a result, as can be confirmed in FIG. 6C, the highest level of neutralizing antibody titer induction was showed in the complexes prepared by the mixing order of mRNA → liposome.

**Sub-conclusion**

In the preparation of mRNA-liposome complexes, there were differences in the *in vivo* expression and immunogenicity inducing ability according to the mixing method, and the highest effect was shown in the mixing in the order of mRNA → liposome.

**Preparation Example 4: Preparation of mRNA-liposome complexes with adjusted mixing orders of liposome, mRNA, and immune booster**

**4-1. mRNA-liposome complex comprising liposome, mRNA, and immune booster**

The immune booster, detoxified lipooligosaccharide (dLOS, TLR-4 agonist; EYEGENE Inc., Republic of Korea) was additionally mixed in Preparation Example 1, thereby preparing mRNA-liposome complexes.

**4-2. Adjustment of mixing order of liposome, mRNA, and immune booster**

The 1 mg/mL of Renilla luciferase mRNA solution stored at -70°C, the 20 mM HEPES buffer (pH 7.4) comprising the liposome solution (LP-DOTAP/DOPE/Chol (40:40:20, w/w/w)), and the 1 mg/mL Renilla luciferase mRNA solution stored at -70°C and the refrigerated liposome solution (LP-DOTAP/DOPE/Chol (40:40:20, w/w/w)) and 20 mM HEPES buffer (pH 7.4) comprising 4% sucrose in Preparation Example 1 were used. Additionally, the immune booster dLOS was used. The samples were mixed by the following six methods, which are the number of combinations of the mixing order of the three solutions (mRNA, liposome, and dLOS) .

a: Liposome → mRNA → dLOS
b: Liposome → dLOS → mRNA
c: mRNA → Liposome → dLOS
d: mRNA → dLOS → Liposome
e: dLOS → Liposome → mRNA
f: dLOS → mRNA → Liposome

Specifically, a 20 mM HEPES buffer (pH 7.4) comprising 4% sucrose was taken in a predetermined amount by using a 200P tip only for RNA and dispensed into microtubes. A first solution was taken in a predetermined amount by using a 200P tip and placed in the microtubes into which the buffer was dispensed, followed by pipetting about 10 times. A second solution was taken in a predetermined amount by using a 200P tip and placed in the microtubes into

which the buffer was dispensed, followed by pipetting about 10 times. A third solution was taken in a predetermined amount by using a 200P tip and placed in the microtubes into which the buffer was dispensed, followed by pipetting about 10 times. Last, the solutions were mixed by pipetting 30 times with a 1000 tip.

**Example 7: mRNA expression according to immune booster content**

In Preparation Example 4-1, liposome-mRNA complexes were prepared by adding the immune booster dLOS with different contents, and then the complexes were examined for the *in vivo* expression level. Particularly, dLOS was added in amount of 0.25 μg, 0.5 μg, 0.75 μg, and 1 μg, and the *in vivo* expression level was examined by the same method as in Example 1.

As a result, as shown in FIG. 7, the expression level was increased when the amount of dLOS added was 0.25 μg to 1 μg.

**Example 8: mRNA expression according to mixing order of liposome, mRNA, and immune booster**

The *in vivo* expression of each of the liposome-mRNA complexes prepared in Preparation Example 4-2 was examined.

As a result, as shown in FIG. 8, a higher expression level was confirmed in the mice administered the composition obtained by mixing in the order of dLOS → mRNA → liposome.

**Example 9: Changes in stability (of physiochemical properties) of mRNA-liposome complexes over time**

[EGFP]

Complexes of EGFP mRNA (SEQ ID NO: 2) and liposome (DOTAP:DOPE:Chol (40:40:20, w/w/w)) were examined for changes in physiochemical properties over time after preparation through DLS measurement.

Specifically, the prepared EGFP mRNA-liposome complexes were measured for size, zeta potential, and PDI while refrigerated in a lyophilized formulation form for 9 weeks.

As a result, as can be confirmed in FIG. 9, the complexes were stable for 9 weeks when the NP ratio was 1.39:1 or lower.

[SARS-CoV-2 S]

Liquid formation and lyophilized formulation of complexes of SARS-CoV-2 S mRNA (SEQ ID NO: 3) and liposome (DOTAP:DOPE:Chol (40:40:20, w/w/w)) were examined for changes in physiochemical properties over time after preparation through DLS measurement.

Specifically, the prepared SARS-CoV-2 S mRNA-liposome complexes were measured for size, zeta potential, and PDI for predetermined periods (0, 2, 4, 8, 12, and 16 weeks) while refrigerated for 16 weeks.

As a result, as can be confirmed in Table 7 (liquid formulation) and Table 8 (lyophilized formulation), both the liquid formulation and the lyophilized formulation were stable for 16 weeks.

Table 7

| Classification | 0 Weeks | 2 Weeks | 4 Weeks | 8 Weeks | 12 Weeks | 16 Weeks |
|---|---|---|---|---|---|---|
| Particle size (nm) | 216.0 | 225.8 | 227.1 | 222.9 | 199.9 | 196.5 |
| Dispersity | 0.187 | 0.194 | 0.175 | 0.219 | 0.187 | 0.159 |
| Zeta potential (mV) | -62.7 | -56.4 | -52.3 | -55.0 | -72.3 | -68.7 |

Table 8

| Classification | 0 Weeks | 2 Weeks | 4 Weeks | 8 Weeks | 12 Weeks | 16 Weeks |
|---|---|---|---|---|---|---|
| Particle size (nm) | 339.1 | 316.1 | 322.4 | 329.2 | 280.5 | 272.0 |
| Dispersity | 0.284 | 0.215 | 0.245 | 0.264 | 0.243 | 0.228 |
| Zeta potential (mV) | -45.4 | -43.7 | -42.8 | -43.8 | -60.7 | -60.2 |

Industrial Applicability

The present disclosure relates to a composition for in vivo delivery of mRNA and, specifically, to a composition for in vivo delivery of mRNA comprising cationic lipid-based liposome and a preparation method therefor.

## Claims

1. A composition for mRNA delivery comprising cationic lipid-based liposome.

2. The composition of claim 1, wherein the liposome further comprises neutral lipid.

3. The composition of claim 2, wherein the liposome further comprise cholesterol.

4. The composition of claim 1, wherein the cationic lipid is at least one selected from the group consisting of dimethyl dioctadecyl ammonium bromide (DDA), 1,2-dioleoyl-3-trimethylammonium propane (DOTAP), 3β-[N-(N',N'-dimethyl aminoethane) carbamoyl cholesterol (DC-Chol), 1,2-dioleoyloxy-3-dimethylammonium propane (DODAP), 1,2-di-O-octadecenyl-3-trimethylammonium propane (DOTMA), 1,2-dimyristoleoyl-sn-glycero-3-ethylphosphocholine (14:1 Etyle PC), 1-palmitoyl-2-oleoyl-sn-glycero-3-ethylphosphocholine (16:0-18:1 Ethyl PC), 1,2-dioleoyl-sn-glycero-3-ethylphosphocholin (18:1 Ethyl PC), 1,2-distearoyl-sn-glycero-3-ethylphosphocholine (18:0 Ethyl PC), 1,2-dipalmitoyl-sn-glycero-3-ethylphosphocholine (16:0 Ethyl PC), 1,2-dimyristoyl-sn-glycero-3-ethylphosphocholine (14:0 Ethyl PC), 1,2-dilauroyl-sn-glycero-3-ethylphosphocholin (12:0 Ethyl PC), N1-[2-((1S)-1-[(3-aminopropyl)amino]-4-[di(3-aminopropyl)amino]butylcarboxamido)ethyl]-3,4-di[oleyloxy]-benzamide (MVL5), 1,2-dimyristoyl-3-dimethylammonium-propane (14:0 DAP), 1,2-dipalmitoyl-3-dimethylammonium-propane (16:0 DAP), 1,2-distearoyl-3-dimethylammonium-propane (18:0 DAP), N-(4-carboxybenzyl)-N,Ndimethyl-2,3-bis(oleoyloxy)propan-1-aminium (DOBAQ), 1,2-stearoyl-3-trimethylammonium-propane (18:0 TAP), 1,2-dipalmitoyl-3-trimethylammonium-propane (16:0 TA), 1,2-dimyristoyl-3-trimethylammonium-propane (14:0 TAP), and N4-cholesteryl-spermine (GL67).

5. The composition of claim 2, wherein the neutral lipid is at least one selected from the group consisting of 1,2-dimyristoyl-sn-glycero-3-phosphorylcholine (DMPC), 1,2-dioleoyl-sn-glycero-3-phosphocholine (DOPC), 1,2-dioleoyl-sn-glycero-3-phosphoethanolamine (DOPE), 1,2-dipalmitoyl-sn-glycero-3-phosphocholine (DPPC), 1,2-distearoyl-sn-glycero-3-phosphocholine (DSPC), 1,2-dilinoleoyl-sn-glycero-3-phosphocholine (DLPC), phosphatidylserine (PS), phosphoethanolamine (PE), phosphatidylglycerol (PG), phosphoric acid (PA), and phosphatidylcholine (PC).

6. The composition of claim 2, wherein the weight ratio of the cationic lipid and the neutral lipid is 1:9-9.5:0.5.

7. The composition of claim 3, wherein the weight ratio of the cationic lipid and the cholesterol is 6:1 - 1:3.

8. The composition of claim 7, wherein the weight ratio of the cationic lipid, the neutral lipid, and the cholesterol is 1-9.5:9-0.5:0.05-3.

9. The composition of claim 1, wherein the mRNA encodes a peptide or protein that may act as an immunogen.

10. The composition of claim 9, wherein the N:P ratio of the liposome and the mRNA is 0.23:1 - 1.39:1.

11. The composition of claim 1, wherein the composition further comprises an immune booster.

12. The composition of claim 11, wherein the immune booster is at least one selected from the group consisting of PAMP, saponin, CpG DNA, lipoprotein, flagella, poly I:C, squalene, tricaprin, 3D-MPL, and detoxied lipooligosaccharide (dLOS).

13. A pharmaceutical composition, comprising the composition for mRNA delivery of any one of claims 1 to 12 as an active ingredient, for prevention or treatment of a disease selected from the group consisting of cancer, tumors, autoimmune diseases, genetic diseases, inflammatory diseases, viral infections, and bacterial infections.

14. A vaccine comprising the composition for mRNA delivery of any one of claims 1 to 12 as an active ingredient.

**15.** A functional cosmetic composition comprising the composition for mRNA delivery of any one of claims 1 to 12 as an active ingredient.

**16.** A method for preparing a composition for mRNA delivery, the method comprising mixing mRNA and liposome.

**17.** The method of claim 16, wherein in the mixing, the mRNA and the liposome are added sequentially.

**18.** The method of claim 16, further comprising mixing an immune booster.

**19.** The method of claim 18, wherein in the mixing, the immune booster, the mRNA, and liposome are added sequentially.

Figure 1

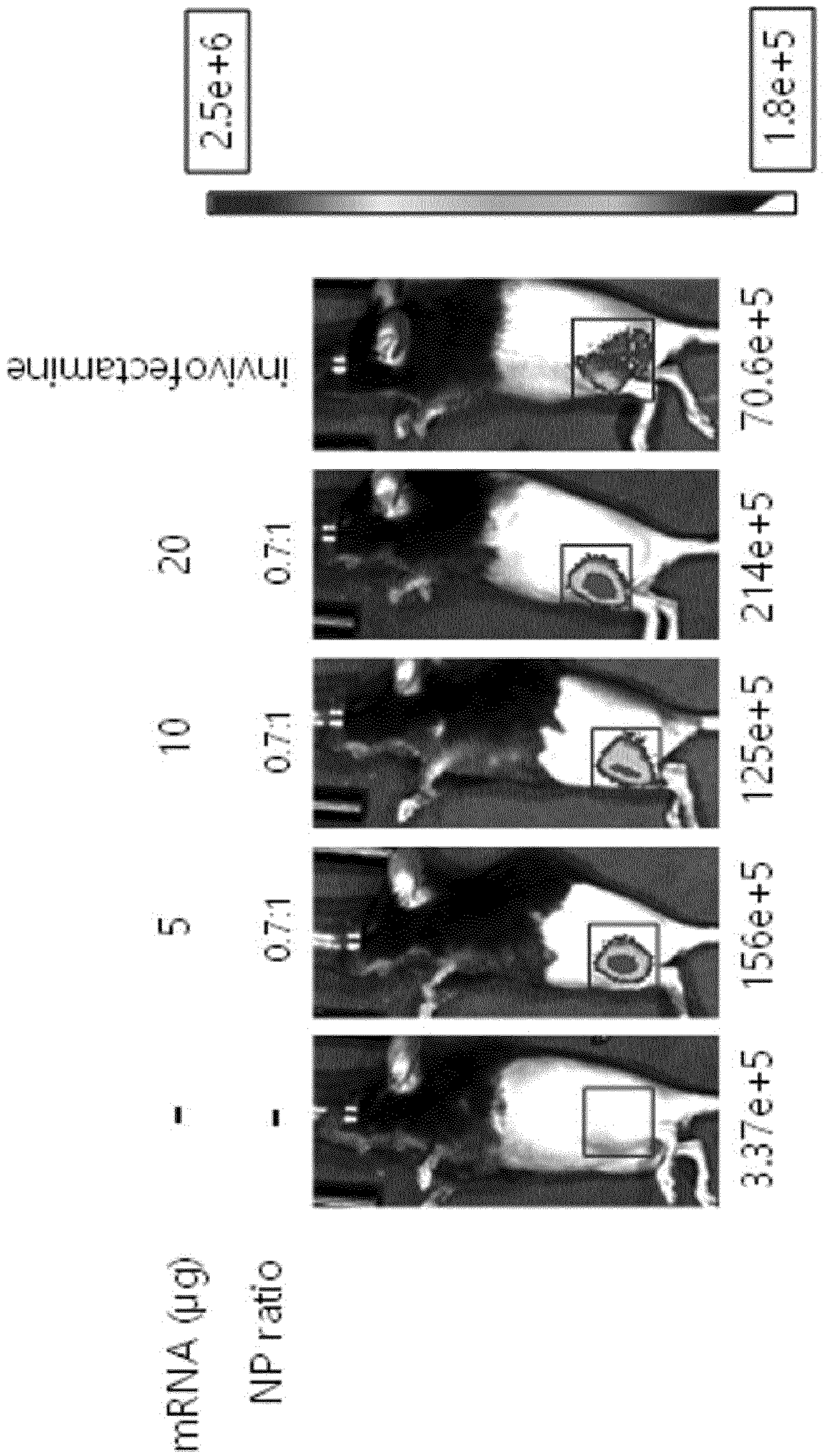

2.5e+6
1.8e+5
mRNA (µg)
NP ratio
-
-
3.37e+5
5
0.7:1
156e+5
10
0.7:1
125e+5
20
0.7:1
214e+5
invivofectamine
70.6e+5

Figure 2

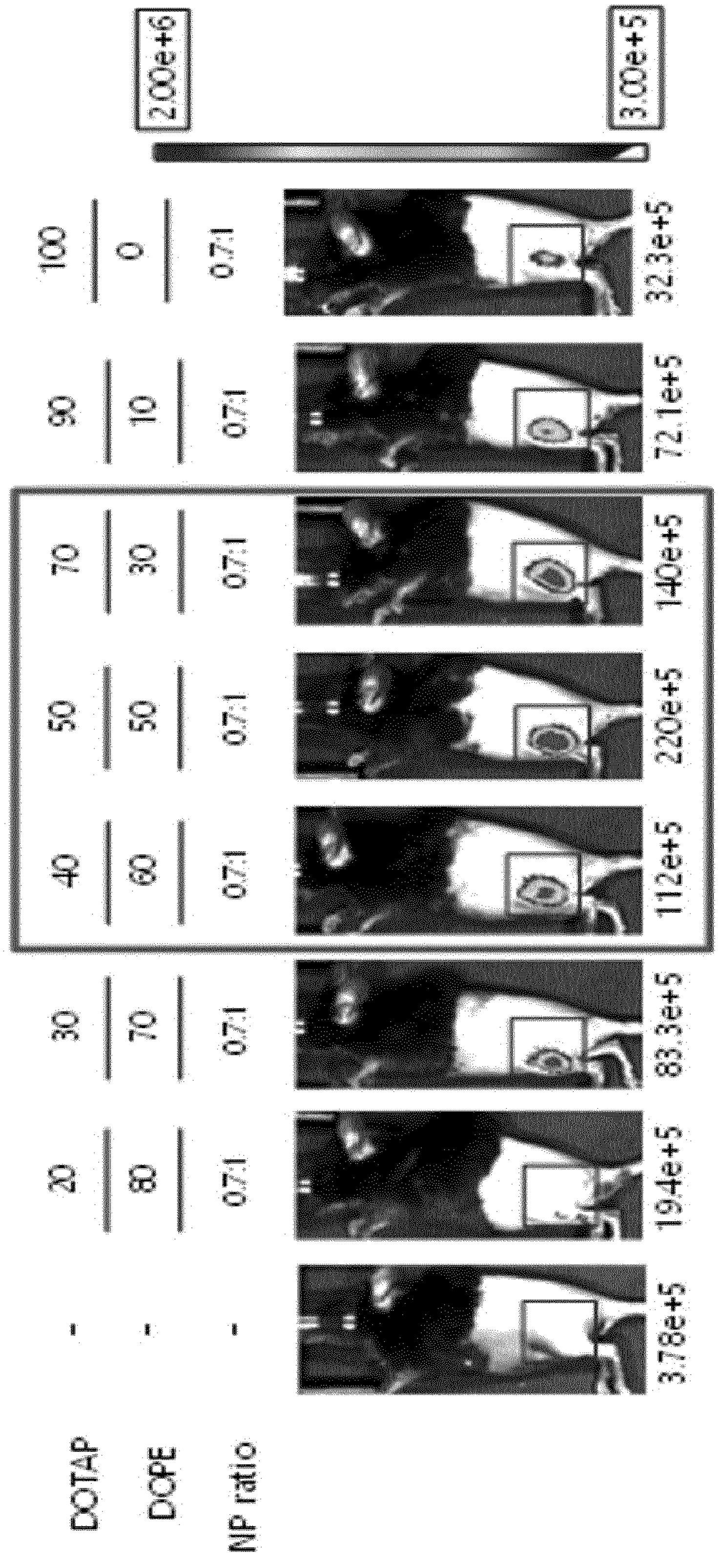
DOTAP
DOPE
NP ratio
-
-
-
3.78e+5
20
80
0.7:1
19.4e+5
30
70
0.7:1
83.3e+5
40
60
0.7:1
112e+5
50
50
0.7:1
220e+5
70
30
0.7:1
140e+5
90
10
0.7:1
72.1e+5
100
0
0.7:1
32.3e+5
2.00e+6
3.00e+5

Figure 3

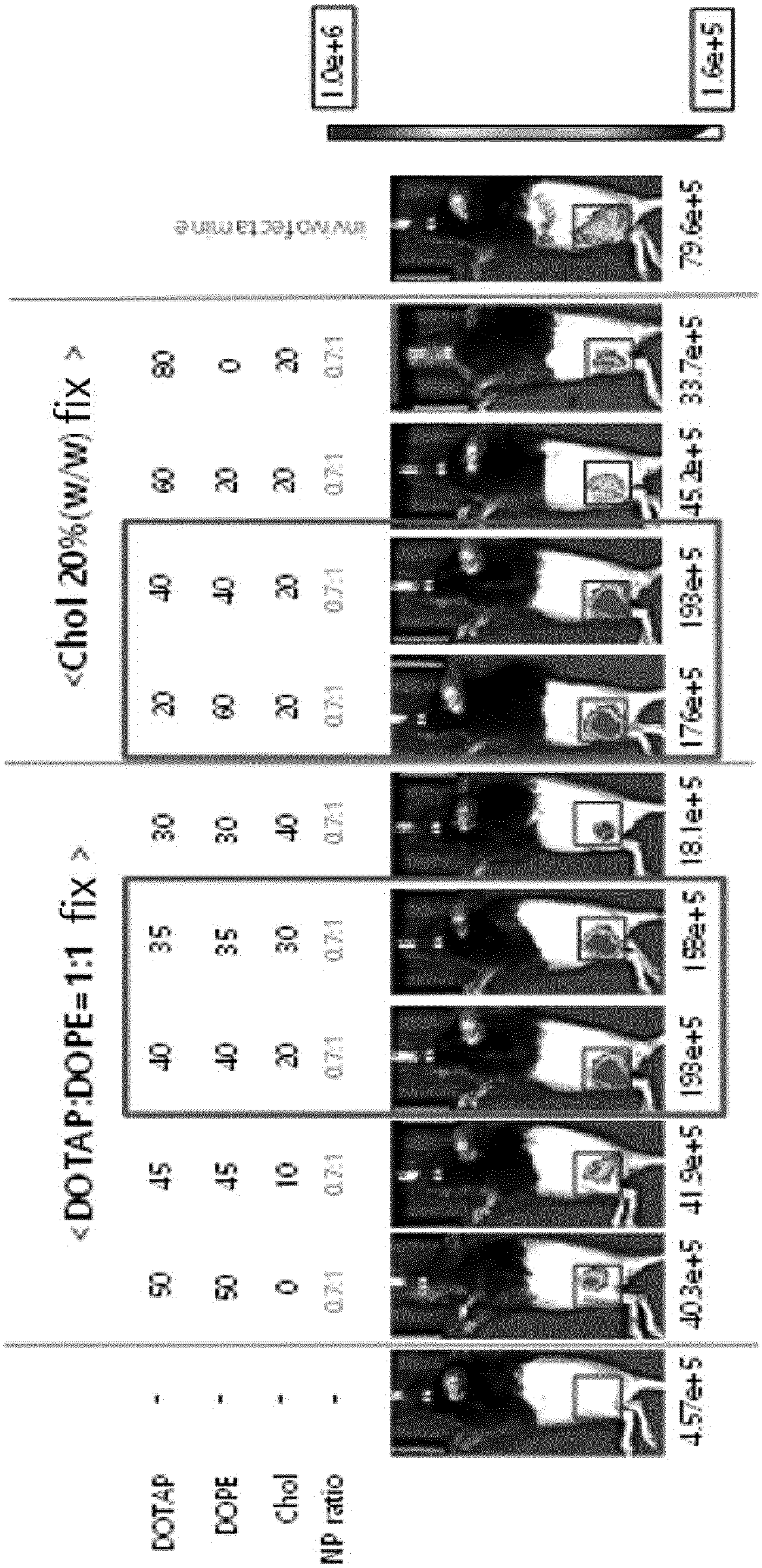
DOTAP
DOPE
Chol
NP ratio
<DOTAP:DOPE=1:1 fix >
<Chol 20%(w/w) fix >
4.57e+5
40.3e+5
41.9e+5
193e+5
198e+5
18.1e+5
176e+5
193e+5
45.3e+5
33.7e+5
79.6e+5
1.0e+6
1.6e+5

Figure 4

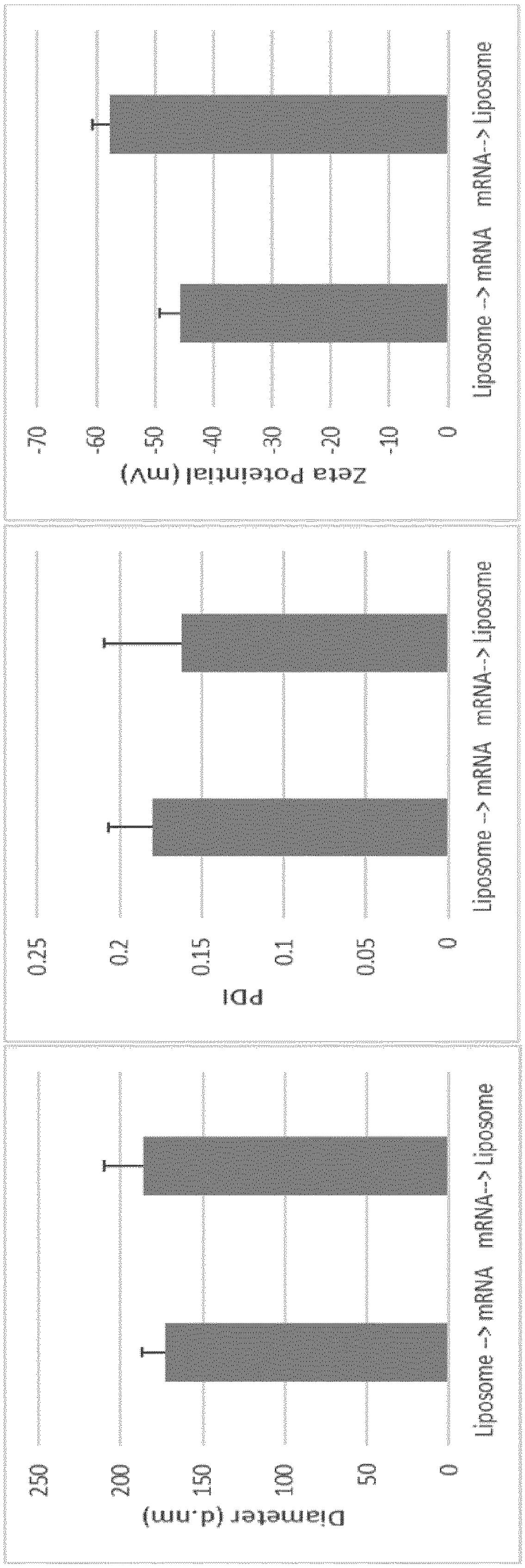
Zeta Poteintial (mV)
-70
-60
-50
-40
-30
-20
-10
0
Liposome --> mRNA
mRNA--> Liposome
PDI
0.25
0.2
0.15
0.1
0.05
0
Liposome --> mRNA
mRNA--> Liposome
Diameter (d.nm)
250
200
150
100
50
0
Liposome --> mRNA
mRNA--> Liposome

Figure 5

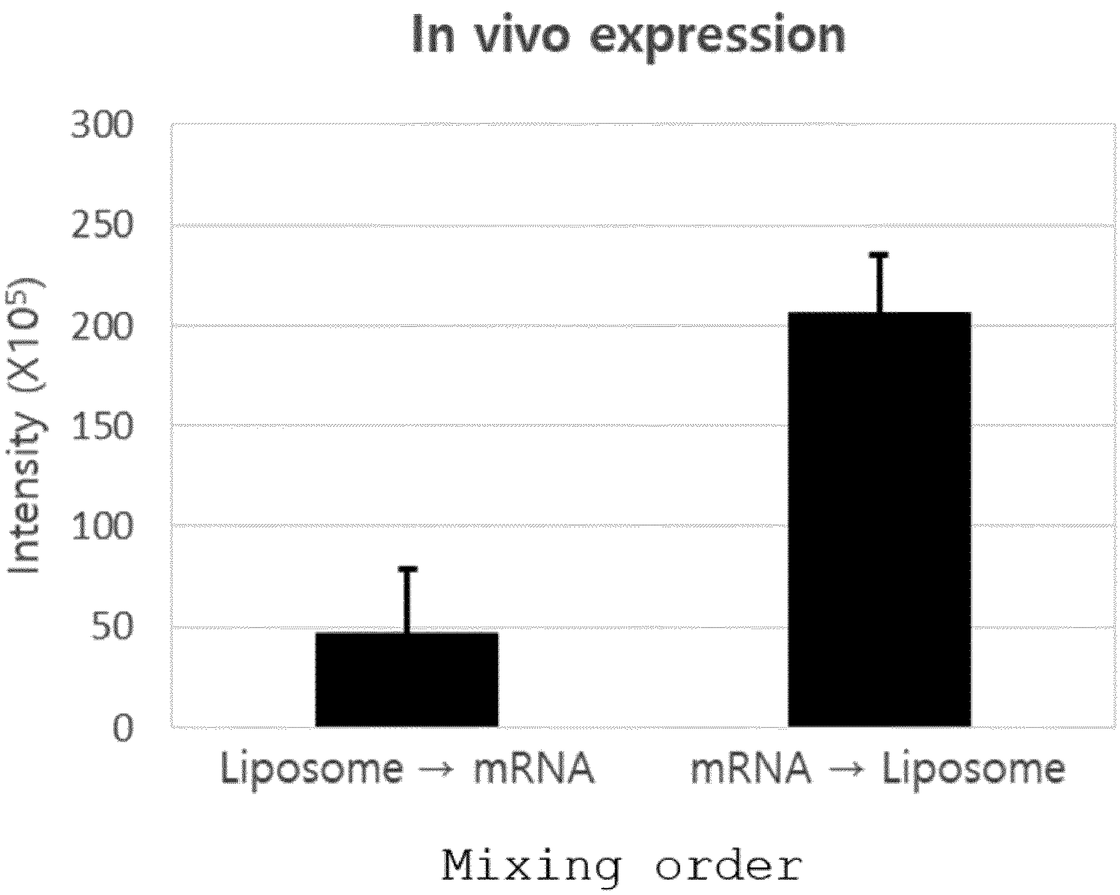
In vivo expression
Intensity (X10^5)
300
250
200
150
100
50
0
Liposome → mRNA
mRNA → Liposome
Mixing order

Figure 6a

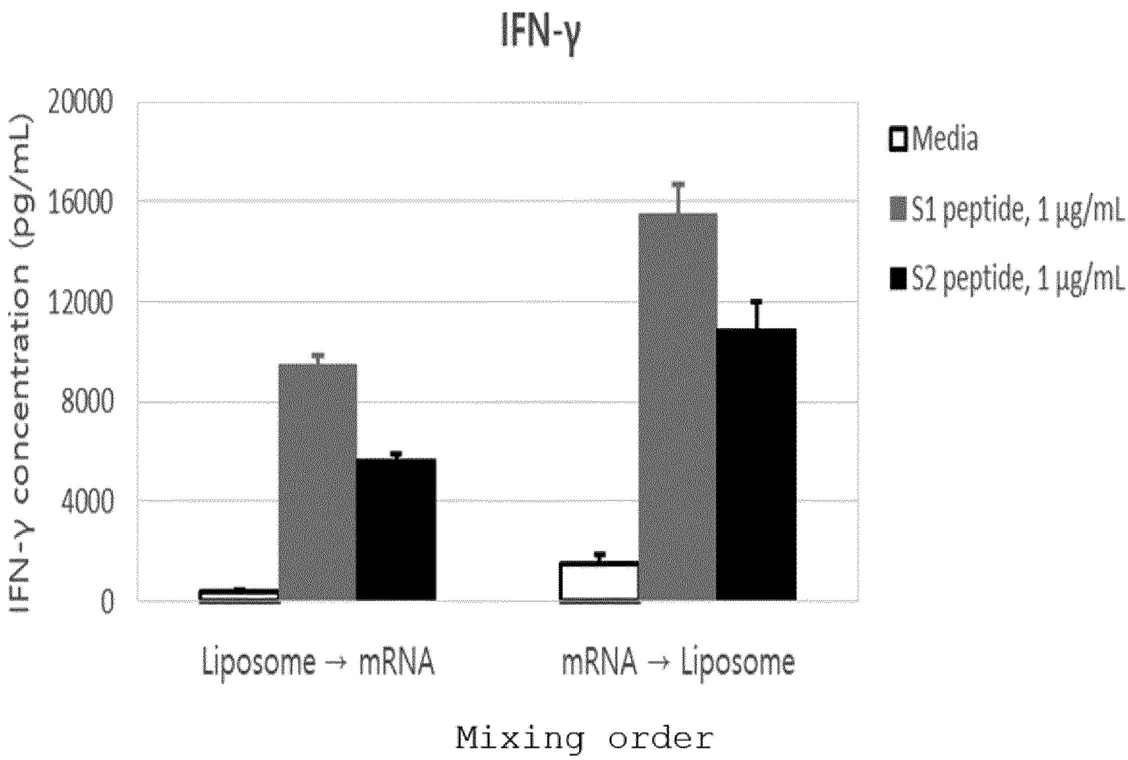
IFN-γ
IFN-γ concentration (pg/mL)
20000
16000
12000
8000
4000
0
Media
S1 peptide, 1 μg/mL
S2 peptide, 1 μg/mL
Liposome → mRNA
mRNA → Liposome
Mixing order

Figure 6b

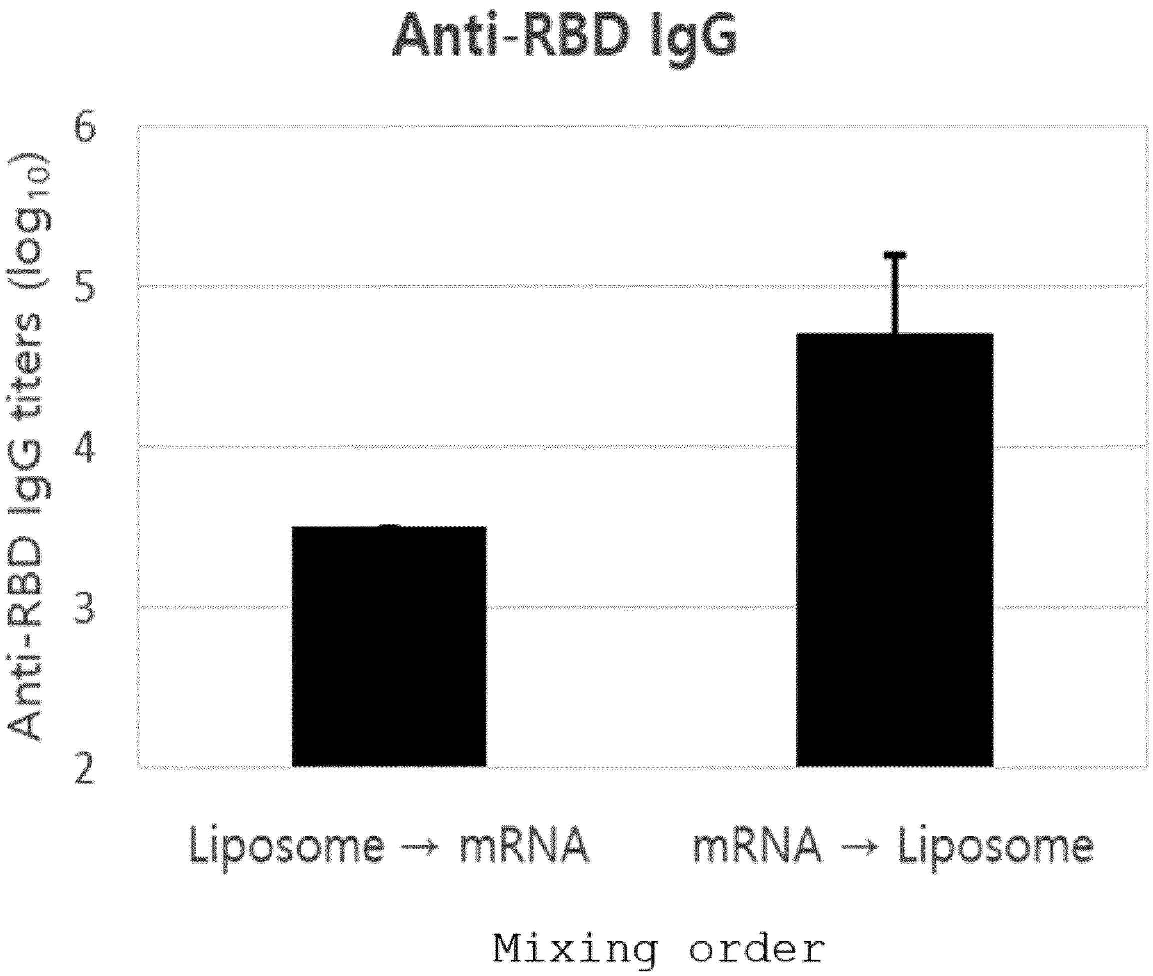
Anti-RBD IgG
Anti-RBD IgG titers ($log_{10}$)
6
5
4
3
2
Liposome → mRNA
mRNA → Liposome
Mixing order

Figure 6c

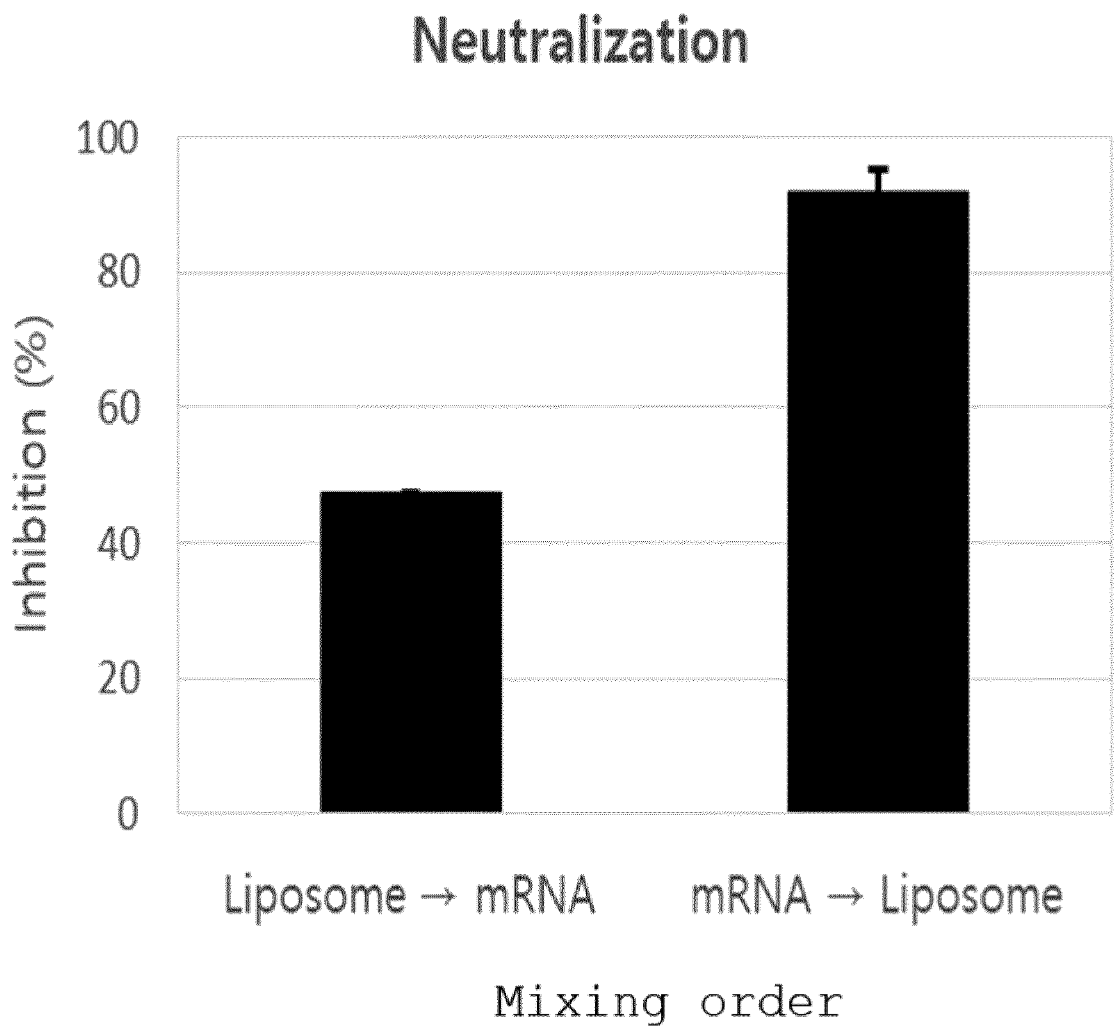
Neutralization
Inhibition (%)
100
80
60
40
20
0
Liposome → mRNA
mRNA → Liposome
Mixing order

Figure 7

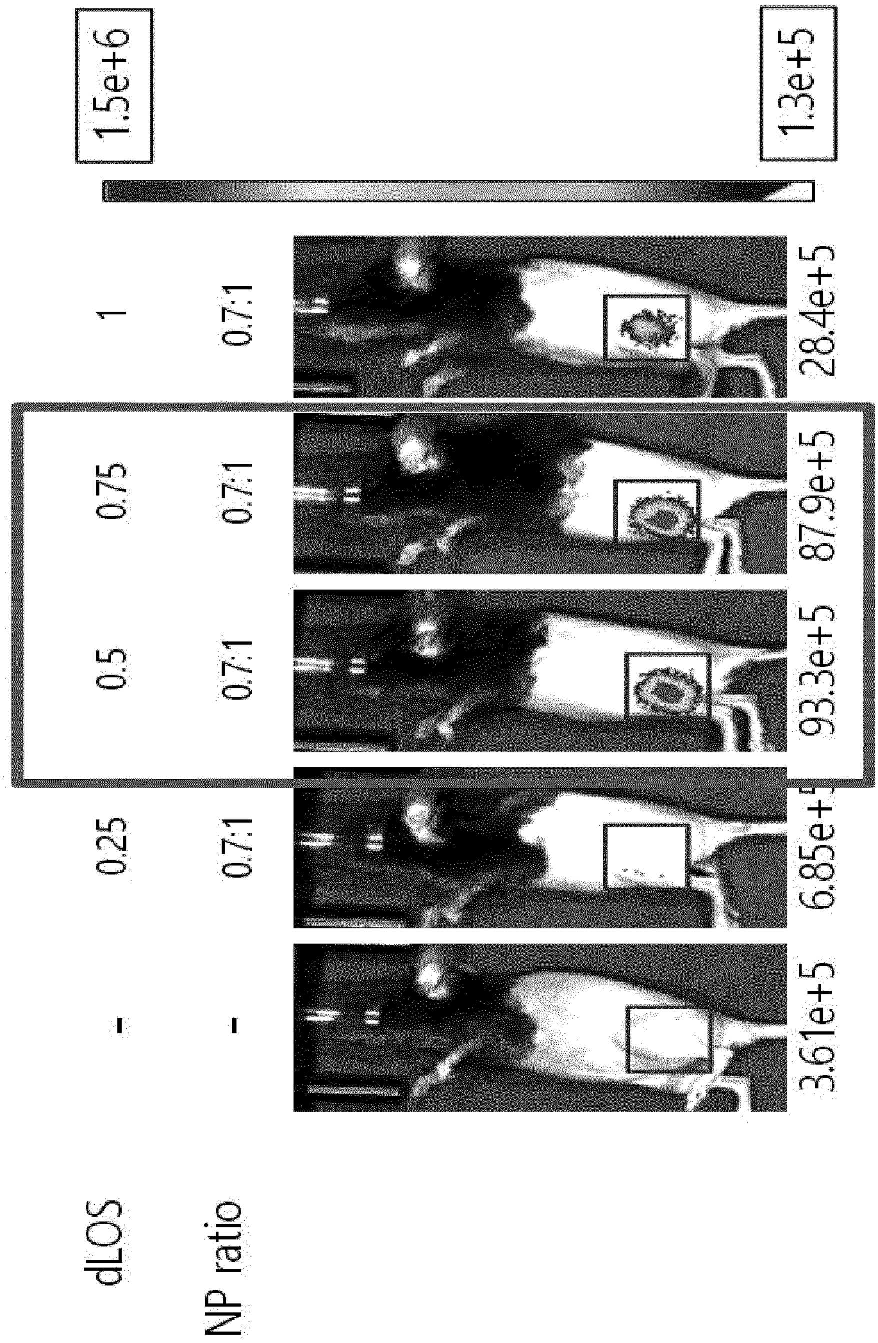
dLOS
NP ratio
-
-
3.61e+5
0.25
0.7:1
6.85e+5
0.5
0.7:1
93.3e+5
0.75
0.7:1
87.9e+5
1
0.7:1
28.4e+5
1.5e+6
1.3e+5

Figure 8

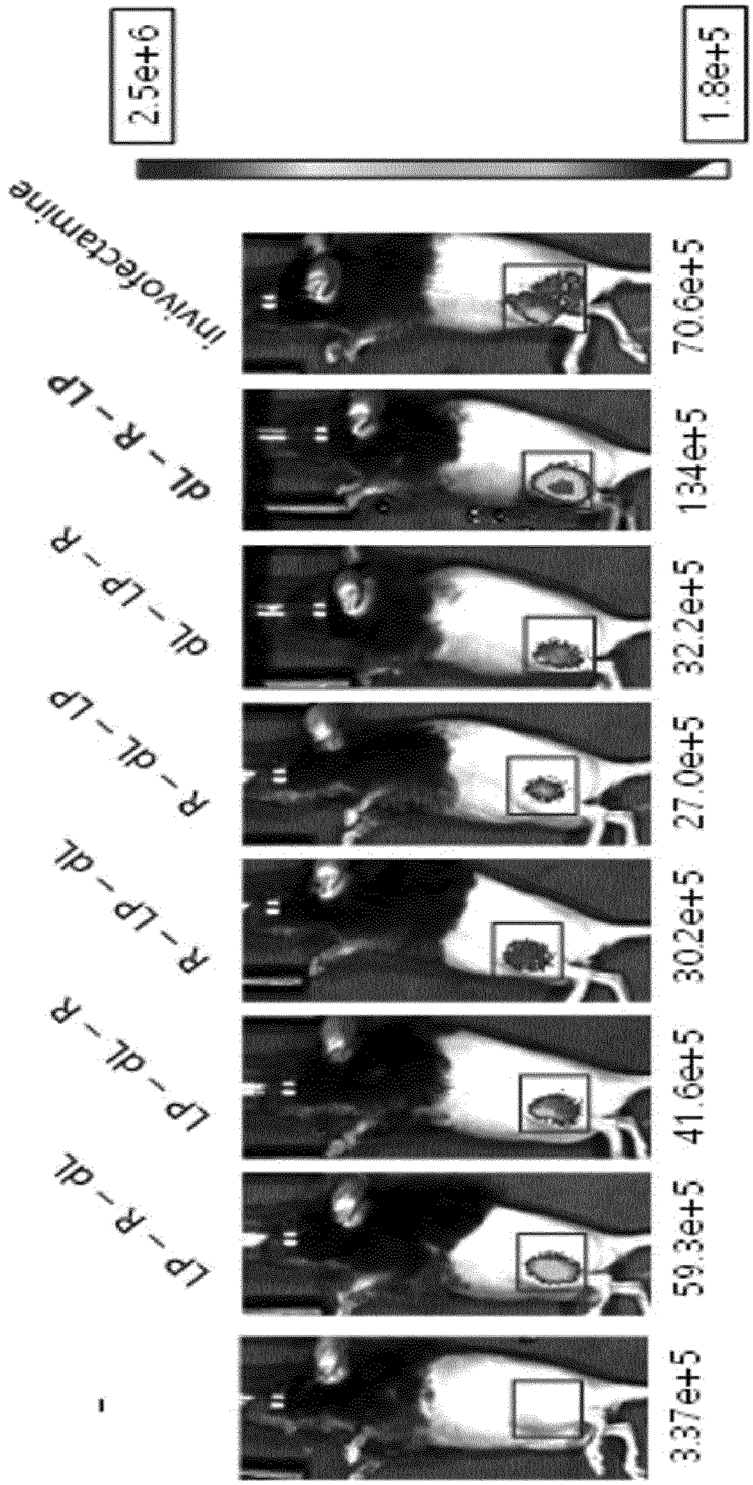
2.5e+6
1.8e+5
invivofectamine
dL – R – Lp
dL – Lp – R
R – dL – Lp
R – Lp – dL
Lp – dL – R
Lp – R – dL
-
70.6e+5
134e+5
32.2e+5
27.0e+5
30.2e+5
41.6e+5
59.3e+5
3.37e+5

Figure 9a

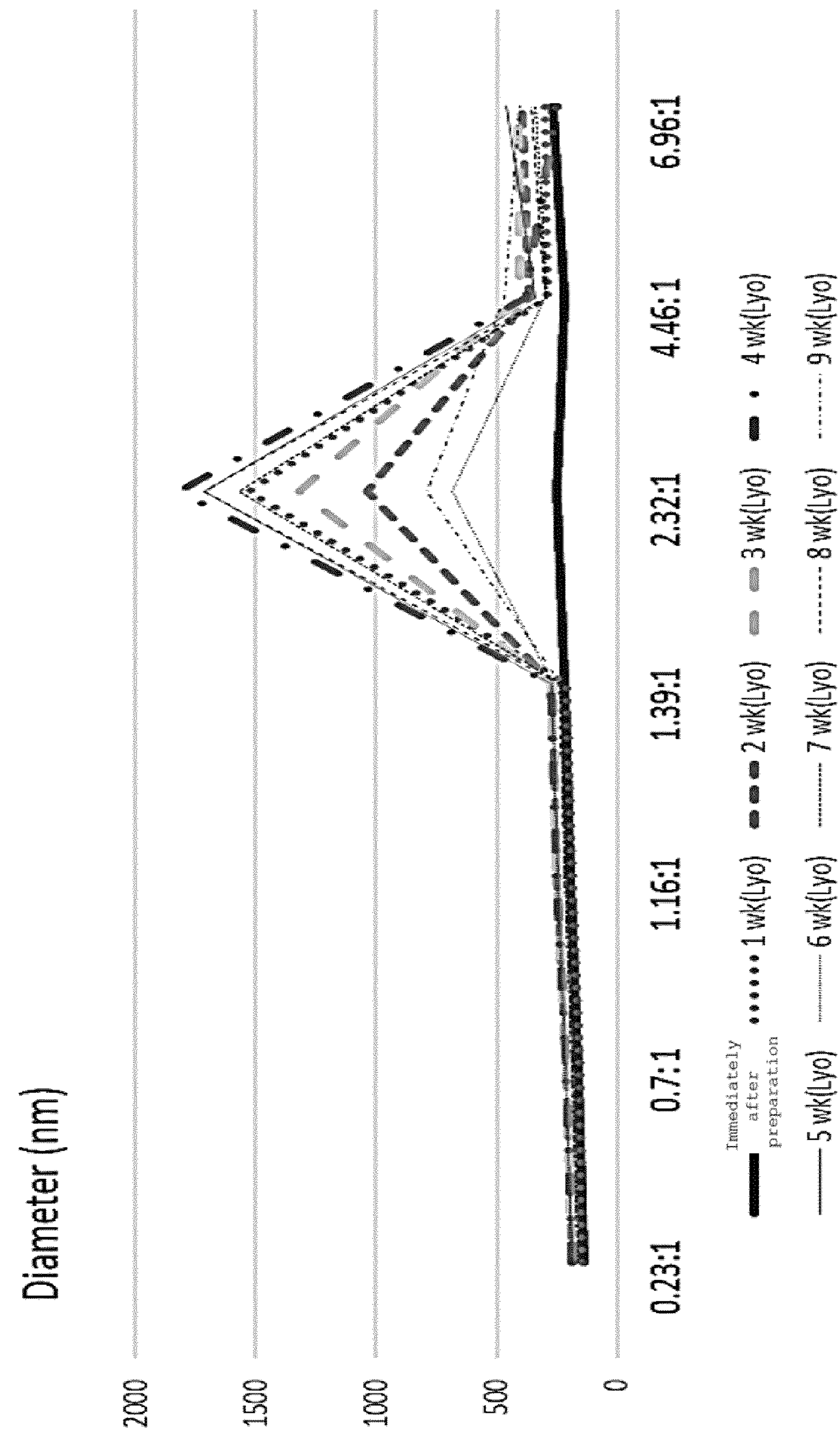
Diameter (nm)
2000
1500
1000
500
0
0.23:1
0.7:1
1.16:1
1.39:1
2.32:1
4.46:1
6.96:1
Immediately after preparation
1 wk(Lyo)
2 wk(Lyo)
3 wk(Lyo)
4 wk(Lyo)
5 wk(Lyo)
6 wk(Lyo)
7 wk(Lyo)
8 wk(Lyo)
9 wk(Lyo)

Figure 9b

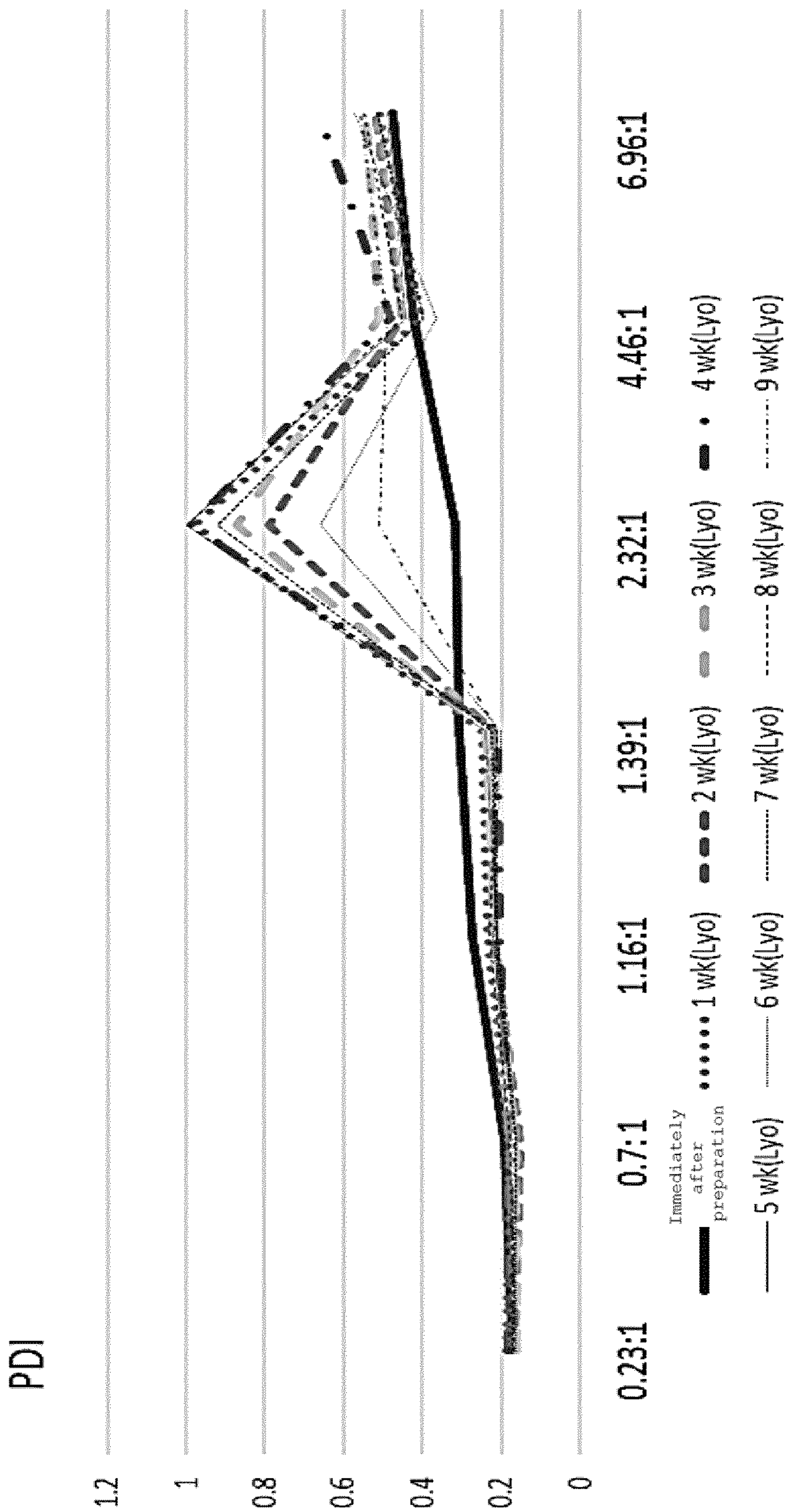
PDI
1.2
1
0.8
0.6
0.4
0.2
0
0.23:1
0.7:1
1.16:1
1.39:1
2.32:1
4.46:1
6.96:1
Immediately after preparation
1 wk(Lyo)
2 wk(Lyo)
3 wk(Lyo)
4 wk(Lyo)
5 wk(Lyo)
6 wk(Lyo)
7 wk(Lyo)
8 wk(Lyo)
9 wk(Lyo)

Figure 9c

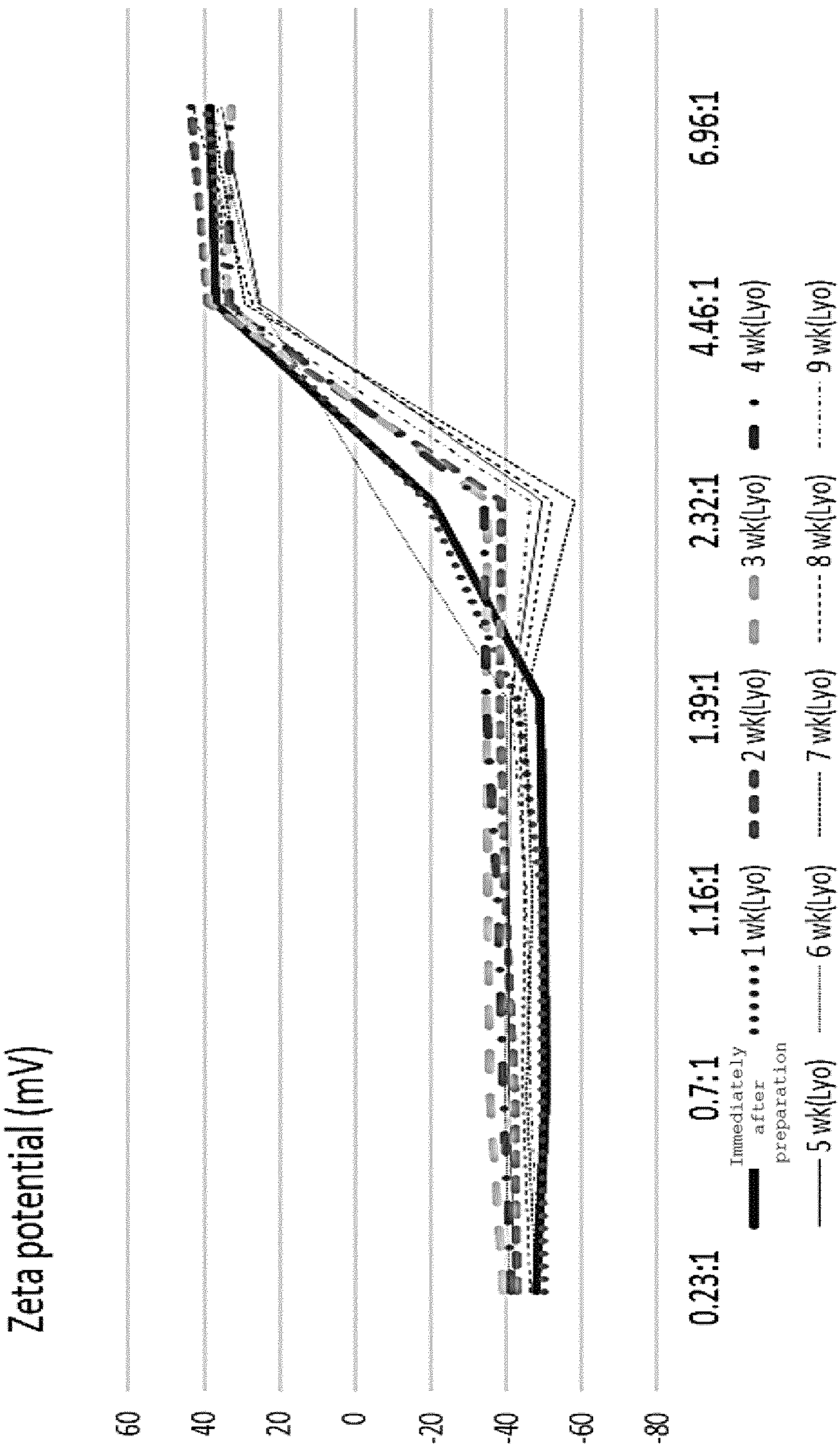
Zeta potential (mV)
60
40
20
0
-20
-40
-60
-80
0.23:1
0.7:1
1.16:1
1.39:1
2.32:1
4.46:1
6.96:1
Immediately after preparation
1 wk(Lyo)
2 wk(Lyo)
3 wk(Lyo)
4 wk(Lyo)
5 wk(Lyo)
6 wk(Lyo)
7 wk(Lyo)
8 wk(Lyo)
9 wk(Lyo)

**INTERNATIONAL SEARCH REPORT**

International application No.
**PCT/KR2022/003220**

**A. CLASSIFICATION OF SUBJECT MATTER**

**A61K 48/00**(2006.01)i; **A61K 9/127**(2006.01)i; **A61K 39/00**(2006.01)i; **A61K 8/14**(2006.01)i; **A61K 8/60**(2006.01)i; **A61Q 19/00**(2006.01)i

According to International Patent Classification (IPC) or to both national classification and IPC

**B. FIELDS SEARCHED**

Minimum documentation searched (classification system followed by classification symbols)

A61K 48/00(2006.01); A61K 38/17(2006.01); A61K 39/00(2006.01); A61K 39/12(2006.01); A61K 47/28(2006.01); A61K 47/50(2017.01); C11B 1/00(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 양이온성 리포좀(cationic liposome), mRNA, 면역반응(immune response), 전달(delivering), 백신(vaccine)

**C. DOCUMENTS CONSIDERED TO BE RELEVANT**

| Category* | Citation of document, with indication, where appropriate, of the relevant passages | Relevant to claim No. |
|---|---|---|
| X | KR 10-2016-0091893 A (SHIRE HUMAN GENETIC THERAPIES, INC. et al.) 03 August 2016 (2016-08-03)<br>See claims 1-13 and 36-49; table 1; and pages 17 and 90. | 1-19 |
| X | KR 10-2001-0069179 A (PARK, Jong Sang) 23 July 2001 (2001-07-23)<br>See abstract; and claims 1-18 and 23-25. | 1-19 |
| X | KR 10-2018-0122128 A (UNIVERSITY INDUSTRY FOUNDATION, YONSEI UNIVERSITY WONJU CAMPUS) 12 November 2018 (2018-11-12)<br>See abstract; claims 1-21; and paragraphs [0034]-[0040]. | 1-19 |
| X | KR 10-2014-0137739 A (APTABIO THERAPEUTICS INC.) 03 December 2014 (2014-12-03)<br>See claims 1-16; and paragraphs [0024]-[0033]. | 1-19 |
| X | KR 10-2019-0021938 A (EYEGENE INC.) 06 March 2019 (2019-03-06)<br>See claims 1-21. | 1-19 |

☐ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **24 June 2022** | **24 June 2022** |
| Name and mailing address of the ISA/KR<br>**Korean Intellectual Property Office**<br>**Government Complex-Daejeon Building 4, 189 Cheongsa-ro, Seo-gu, Daejeon 35208**<br>Facsimile No. **+82-42-481-8578** | Authorized officer<br><br>Telephone No. |

Form PCT/ISA/210 (second sheet) (July 2019)

**INTERNATIONAL SEARCH REPORT**

International application No.
**PCT/KR2022/003220**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed:
      ☑ in the form of an Annex C/ST.25 text file.
      ☐ on paper or in the form of an image file.
   b. ☐ furnished together with the international application under PCT Rule 13*ter*.1(a) for the purposes of international search only in the form of an Annex C/ST.25 text file.
   c. ☐ furnished subsequent to the international filing date for the purposes of international search only:
      ☐ in the form of an Annex C/ST.25 text file (Rule 13*ter*.1(a)).
      ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

Form PCT/ISA/210 (continuation of first sheet) (July 2019)

**INTERNATIONAL SEARCH REPORT**
**Information on patent family members**

International application No.
**PCT/KR2022/003220**

| Patent document cited in search report | Publication date (day/month/year) | Patent family member(s) | Publication date (day/month/year) |
|---|---|---|---|
| KR 10-2016-0091893 A | 03 August 2016 | AU 2014-340155 A1 | 05 May 2016 |
| | | AU 2016-340155 A1 | 05 May 2016 |
| | | CA 2928078 A1 | 30 April 2015 |
| | | CL 2016000957 A1 | 09 December 2016 |
| | | CN 105813656 A | 27 July 2016 |
| | | CN 105813656 B | 15 January 2021 |
| | | CN 112618732 A | 09 April 2021 |
| | | CN 112656954 A | 16 April 2021 |
| | | EA 201690576 A1 | 31 October 2016 |
| | | EP 3060257 A1 | 31 August 2016 |
| | | EP 3060257 B1 | 24 February 2021 |
| | | EP 3871696 A1 | 01 September 2021 |
| | | ES 2865699 T3 | 15 October 2021 |
| | | JP 2016-535738 A | 17 November 2016 |
| | | JP 2019-073557 A | 16 May 2019 |
| | | JP 2020-073567 A | 14 May 2020 |
| | | JP 2021-091729 A | 17 June 2021 |
| | | JP 6525435 B2 | 12 June 2019 |
| | | JP 6646773 B2 | 14 February 2020 |
| | | JP 6851516 B2 | 31 March 2021 |
| | | JP 7046246 B2 | 01 April 2022 |
| | | KR 10-1988552 B1 | 25 September 2019 |
| | | KR 10-2019-0067261 A | 14 June 2019 |
| | | KR 10-2096796 B1 | 27 May 2020 |
| | | MX 2016005238 A | 12 August 2016 |
| | | SG 11201602943 PA | 30 May 2016 |
| | | US 10052284 B2 | 21 August 2018 |
| | | US 10493031 B2 | 03 December 2019 |
| | | US 10959953 B2 | 30 March 2021 |
| | | US 2015-0140070 A1 | 21 May 2015 |
| | | US 2017-0281542 A1 | 05 October 2017 |
| | | US 2018-0369144 A1 | 27 December 2018 |
| | | US 2020-0078299 A1 | 12 March 2020 |
| | | US 9629804 B2 | 25 April 2017 |
| | | WO 2015-061467 A1 | 30 April 2015 |
| KR 10-2001-0069179 A | 23 July 2001 | AU 2000-51102 A1 | 18 December 2000 |
| | | AU 5110200 A | 18 December 2000 |
| | | EP 1185674 A1 | 13 March 2002 |
| | | JP 2003-501363 A | 14 January 2003 |
| | | KR 10-0454881 B1 | 05 November 2004 |
| | | KR 10-1999-0068514 A | 06 September 1999 |
| | | KR 10-2002-0013528 A | 20 February 2002 |
| | | WO 00-73471 A1 | 07 December 2000 |
| KR 10-2018-0122128 A | 12 November 2018 | KR 10-1979497 B1 | 16 May 2019 |
| KR 10-2014-0137739 A | 03 December 2014 | KR 10-2008787 B1 | 08 August 2019 |
| KR 10-2019-0021938 A | 06 March 2019 | AU 2017-351899 A1 | 06 June 2019 |
| | | AU 2017-351900 A1 | 06 June 2019 |
| | | CN 110461355 A | 15 November 2019 |
| | | CN 110545840 A | 06 December 2019 |
| | | EA 201991076 A1 | 31 October 2019 |

Form PCT/ISA/210 (patent family annex) (July 2019)

**INTERNATIONAL SEARCH REPORT**
**Information on patent family members**

International application No.
**PCT/KR2022/003220**

| Patent document cited in search report | Publication date (day/month/year) | Patent family member(s) | Publication date (day/month/year) |
|---|---|---|---|
| | | EA 201991081 A1 | 31 October 2019 |
| | | EP 3533463 A1 | 04 September 2019 |
| | | EP 3533464 A1 | 04 September 2019 |
| | | JP 2020-500213 A | 09 January 2020 |
| | | JP 2020-502258 A | 23 January 2020 |
| | | JP 6963018 B2 | 05 November 2021 |
| | | JP 6963019 B2 | 05 November 2021 |
| | | KR 10-2018-0047054 A | 10 May 2018 |
| | | KR 10-2019-0000586 A | 03 January 2019 |
| | | KR 10-2019-0021939 A | 06 March 2019 |
| | | KR 10-2042993 B1 | 11 November 2019 |
| | | KR 10-2086986 B1 | 10 March 2020 |
| | | KR 10-2086988 B1 | 10 March 2020 |
| | | US 10874733 B2 | 29 December 2020 |
| | | US 10918709 B2 | 16 February 2021 |
| | | US 2019-0255171 A1 | 22 August 2019 |
| | | US 2019-0290749 A1 | 26 September 2019 |
| | | WO 2018-080252 A1 | 03 May 2018 |
| | | WO 2018-080253 A1 | 03 May 2018 |

Form PCT/ISA/210 (patent family annex) (July 2019)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- KR 1020210029929
- KR 1509456
- KR 2042993
- CH 2900014, Merck & Cie

### Non-patent literature cited in the description


- **WOLFF JA et al.** *Science,* 1990, vol. 247, 1465-8
- **SAYOUR EJ et al.** *J Immunother Cancer,* 2015, vol. 3, 13